# EUROPEAN PATENT APPLICATION

(11) **EP 4 385 673 A1**
(43) Date of publication of application: **19.06.2024**
(21) Application number: 22878701.6
(22) Date of filing: 09.08.2022
(51) Int. Cl.: B25J 9/00, B25J 9/12, B25J 9/16, A61H 1/02, A61H 37/00, A63B 23/04, A63B 21/00, G08B 3/00, H04R 1/32

(54) **ELECTRONIC DEVICE FOR CONTROLLING ACTUATOR ON BASIS OF ROTARY MOTION OF JOINT, AND METHOD FOR SAME**

(30) Priority: 05.10.2021 KR 20210132004; 19.10.2021 KR 20210139744
(71) Applicant: Samsung Electronics Co., Ltd., Suwon-si, Gyeonggi-do 16677 (KR)
(72) Inventor: HWANG, Jiwoong, Suwon-si, Gyeonggi-do 16677 (KR); LEE, Seungjoon, Suwon-si, Gyeonggi-do 16677 (KR); HAN, Hoon, Suwon-si, Gyeonggi-do 16677 (KR)
(74) Representative: Gulde & Partner
(86) International application number: PCT/KR2022/011882
(87) International publication number: WO 2023/058880

(57) **Abstract**

An electronic device according to an embodiment comprises: an actuator related to a joint of a user of the electronic device; at least one sensor; memory for storing one or more instructions; and a processor operatively coupled to the actuator, the at least one sensor, and the memory. The processor, when executing the one or more instructions, can: use the actuator to adjust the strength of the rotary motion of the joint to a first strength indicated by information related to the user; use the at least one sensor to identify data representing the rotary motion of the joint after adjusting the strength of the rotary motion to the first strength; and, on the basis of the identified data, adjust the strength of the rotary motion from the first strength to a second strength different from the first strength at least on the basis of identifying that the rotary motion has stopped for a designated duration.

## Description

### [Technical Field]

The following descriptions relate to an electronic device and a method for controlling an actuator based on rotational motion of a joint.

### [Background Art]

Recently, due to advancements in electronic technology, a user-wearable electronic device is being developed. For example, the electronic device may operate to assist a user's motion, such as walking. Since a type of the motion capable of being performed by the user is not limited to the walking, a method for assisting the user's other motion different from walking by using the electronic device may be required.

### [Disclosure]

### [Technical Problem]

Among motions performed by an electronic device by a user, a method for assisting the strength exercise may be required.

The technical problems to be achieved in this document are not limited to those described above, and other technical problems not mentioned herein will be clearly understood by those having ordinary knowledge in the art to which the present disclosure belongs, from the following description.

### [Technical Solution]

According to an embodiment, an electronic device may comprise an actuator associated with a joint of a user of the electronic device, at least one sensor, memory storing one or more instructions, and a processor operatively coupled with the actuator, the at least one sensor, and the memory. The processor may be configured to, when executing the one or more instructions, adjust strength of rotational motion of the joint as first strength indicated by information associated with the user by using the actuator. The processor may be configured to, when executing the one or more instructions, after adjusting the strength of the rotational motion to the first strength, identify data indicating the rotational motion of the joint by using the at least one sensor. The processor may be configured to, when executing the one or more instructions, adjust the strength of the rotational motion from the first strength to second strength different from the first strength, at least based on identifying the rotational motion is ceased for a preset duration based on the identified data.

As described above, according to an embodiment, a method of an electronic device may identifying rotational motion rotated along a first direction at a joint of a user of the electronic device, by using at least one sensor included in the electronic device. The method may comprise, in response to identifying the rotational motion of the j oint, outputting torque having first strength and based on a second direction different from the first direction, by using an actuator included in the electronic device and corresponding to the joint. The method may comprise, while outputting the torque by using the actuator, identifying data indicating the rotational motion changed by the torque by using the at least one sensor. The method may comprise changing strength of the torque to second strength lower than the first strength by using the actuator, at least based on identifying the data indicating the rotational motion maintained within a first range during a first time section. The method may comprise changing a direction of the torque to a direction based on the first direction among the first direction and the second direction, by using the actuator, at least based on identifying the data indicating the rotational motion that is maintained within a second range during a second time section while outputting the torque based on the second direction and having the second strength.

As described above, according to an embodiment, a method of an electronic device may comprise adjusting strength of rotational motion of the joint as first strength indicated by information associated with the user by using an actuator associated with a joint of a user of the electronic device. The method may comprise, after adjusting the strength of the rotational motion to the first strength, identifying data indicating the rotational motion of the joint by using the at least one sensor included in the electronic device. The method may comprise at least based on identifying the rotational motion is ceased for a preset duration based on the identified data, adjusting the strength of the rotational motion from the first strength to second strength different from the first strength.

As described above, according to an embodiment, an electronic device may comprise an actuator associated with a joint of a user of the electronic device, at least one sensor, memory storing one or more instructions, and a processor operatively coupled with the actuator, the at least one sensor, and the memory. The processor may be configured to, when executing the one or more instructions, by using the actuator least one sensor included in the electronic device, identify rotational motion rotated along a first direction at a joint of a user of the electronic device. The processor may be configured to, when executing the one or more instructions, in response to identifying the rotational motion of the joint, output torque having first strength and based on a second direction different from the first direction, by using the actuator. The processor may be configured to, when executing the one or more instructions, while outputting the torque by using the actuator, identify data indicating the rotational motion changed by the torque by using the at least one sensor. The processor may be configured to, when executing the one or more instructions, at least based on identifying the data indicating the rotational motion maintained within a first range during a first time section, change strength of the torque to second strength lower than the first strength by using the actuator. The processor may be configured to, when executing the one or more instructions, at least based on identifying the data indicating the rotational motion that is maintained within a second range during a second time section while outputting the torque based on the second direction and having the second strength, change a direction of the torque to a direction based on the first direction among the first direction and the second direction, by using the actuator.

### [Advantageous Effects]

According to an embodiment, an electronic device can increase the quantity of motion according to a user's strength exercise performed based on a joint by using an actuator corresponding to the user's joint.

According to an embodiment, the electronic device can monitor the strength exercise performed by the user based on one or more parameters associated with the joint.

According to an embodiment, the electronic device can guide motion for completing the strength exercise by using the actuator, when the user reaches a limit of the strength exercise.

The effects that can be obtained from the present disclosure are not limited to those described above, and any other effects not mentioned herein will be clearly understood by those having ordinary knowledge in the art to which the present disclosure belongs, from the following description.

### [Description of the Drawings]

FIG. 1 is a diagram illustrating an embodiment of an electronic device in a network environment.
FIG. 2 is an exemplary diagram for explaining a structure of an electronic device, according to an embodiment.
FIG. 3 is an exemplary state transitional diagram of an electronic device, according to an embodiment.
FIG. 4 is a diagram illustrating an example of a user interface (UI) displayed by an external electronic device associated with an electronic device, according to an embodiment.
FIG. 5 is an exemplary diagram for illustrating an operation performed by an electronic device according to a user's motion, according to an embodiment.
FIG. 6 is an exemplary diagram for illustrating another operation performed by an electronic device according to a user's motion, according to an embodiment.
FIG. 7 is an exemplary diagram for illustrating still another operation performed by an electronic device according to a user's motion, according to an embodiment.
FIG. 8 is an exemplary diagram for illustrating an operation in which an electronic device cease to output torque by using an actuator, according to an embodiment.
FIG. 9 is a flowchart illustrating an operation performed by an electronic device according to an embodiment.
FIG. 10 is a flowchart illustrating an operation performed by an electronic device to increase a user's quantity of motion by rotational motion, according to an embodiment.
FIG. 11 is a flowchart illustrating an operation performed by an electronic device to maintain a user's rotational motion, according to an embodiment.
FIG. 12 is a flowchart illustrating an operation performed by an electronic device to guide a user's rotational motion, according to an embodiment.
FIG. 13 is a flowchart illustrating an operation in which an electronic device ceases outputting a torque using an actuator, according to an embodiment.

### [Mode for Invention]

Hereinafter, various embodiments of the present document will be described with reference to the accompanying drawings.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

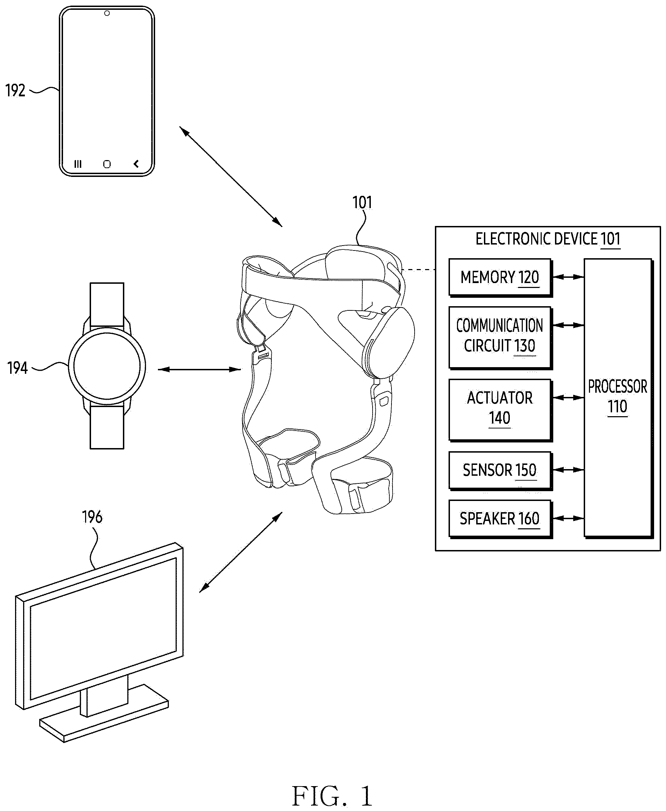

FIG. 1 is a diagram illustrating an embodiment of an electronic device 101 in a network environment. Referring to FIG. 1, an exemplary situation in which the electronic device 101 is connected to one or more external electronic devices (e.g., external electronic devices 192, 194, and 196) based on a wired network and/or a wireless network is illustrated. The wired network may include networks such as Internet, Local Area Network (LAN), Wide Area Network (WAN), Ethernet, or a combination thereof. The wireless network may include networks such as Long Term Evolution (LTE), 5g New Radio (NR), Wireless Fidelity (Wi-Fi), Zigbee, Near Field Communication (NFC), Bluetooth, Bluetooth Low-Energy (BLE), or a combination thereof. Although the electronic device 101 is shown to be directly connected to the external electronic devices 192, 194, and 196, the electronic device 101 may be indirectly connected to at least one of the external electronic devices 192, 194, and 196 through one or more routers and/or Access Point (AP).

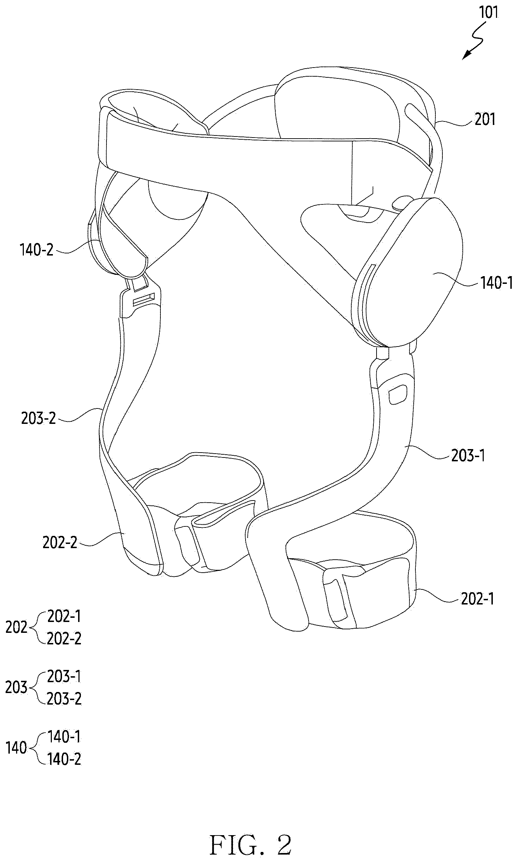

Referring to FIG. 1, according to an embodiment, the electronic device 101 may have a form-factor wearable by a user. For example, the electronic device 101 may include a member attachable to one or more joints of the user. A structure of the electronic device 101 having a wearable form-factor will be described later with reference to FIG. 2.

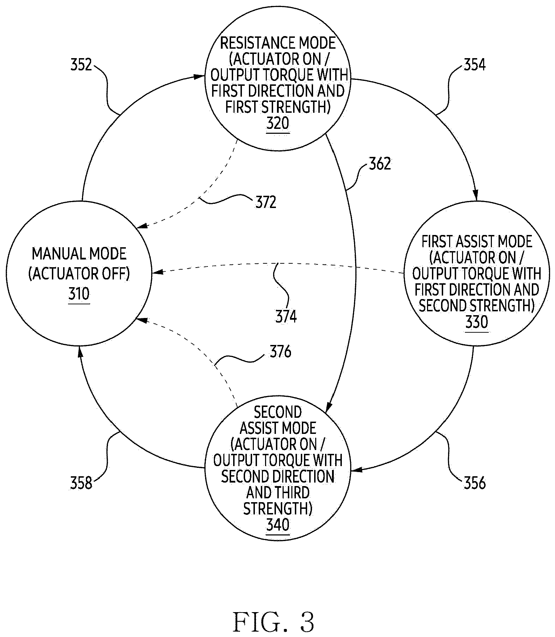

Referring to FIG. 1, external electronic devices 192, 194, and 196 connectable to the electronic device 101 may be a terminal owned by the user. For example, the terminal may correspond to a smartphone, a smart pad, and/or a tablet PC, like as the external electronic device 192. For example, the terminal may correspond to a smart accessory such as a smart watch and/or a head-mounted device (HMD), like as the external electronic device 194. For example, the terminal may correspond to an electronic device including a display such as a monitor, a television, and/or a personal computer (PC), like as the external electronic device 196. According to an embodiment, an operation performed by the electronic device 101 in a state in which the electronic device 101 establishes a connection with one or more external electronic devices 192, 194, and 196 will be described in detail with reference to FIGS. 3 to 13.

Referring to FIG. 1, a block diagram illustrating a hardware component included in the electronic device 101 according to an embodiment is illustrated. The electronic device 101 according to an embodiment may include at least one of a processor 110, a memory 120, a communication circuit 130, an actuator 140, a sensor 150, and a speaker 160. The processor 110, the memory 120, the communication circuit 130, the actuator 140, the sensor 150, and the speaker 160 may be electronically and/or operably coupled with each other by an electronical component such as a communication bus. A type and/or number of hardware components included in the electronic device 101 is not limited as illustrated in FIG. 1. For example, the electronic device 101 may include only some of the hardware components illustrated in FIG. 1. For example, the number of the actuator 140 included in the electronic device 101 may be one or more.

The processor 110 of the electronic device 101 according to an embodiment may include a hardware component for processing data based on one or more instructions. The hardware components for processing data may include, for example, Arithmetic and Logic Unit (ALU), Field Programmable Gate Array (FPGA), and/or a Central Processing Unit (CPU). The number of processors 110 may be one or more. For example, the processor 110 may have a structure of a multi-core processor such as a dual core, a quad core, or a hexa core.

The memory 120 of the electronic device 101 according to an embodiment may include a hardware component for storing data and/or instructions inputted and/or outputted to the processor 110. For example, the memory 120 may include Volatile Memory such as Random-Access Memory (RAM) and/or Non-Volatile Memory such as Read-Only Memory (ROM). For example, the volatile memory may include at least one of dynamic RAM (DRAM), Static RAM (SRAM), Cache RAM, and Pseudo SRAM (PSRAM). For example, the non-volatile memory may include at least one of Programmable ROM (PROM), Electrically Erasable PROM (EEPROM), flash memory, hard disk, compact disk, and Embedded Multi-Media Card (EMMC).

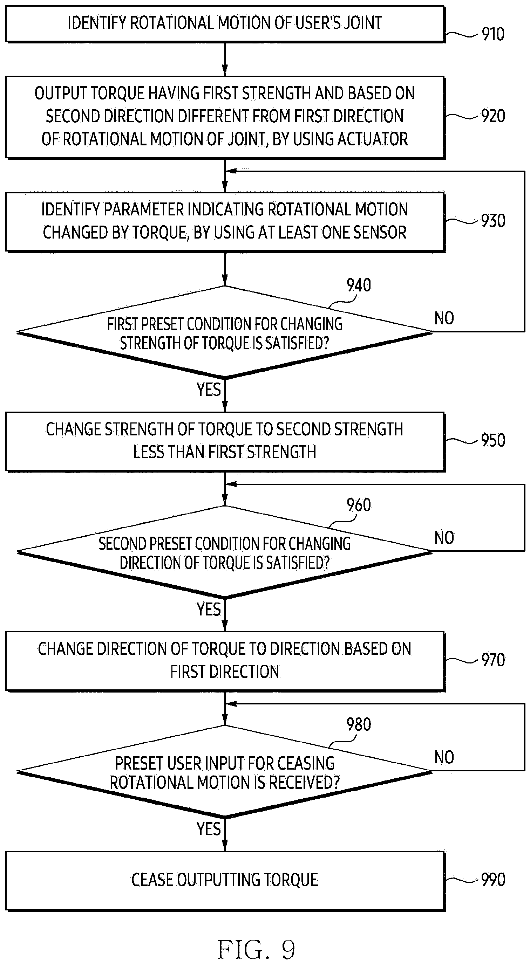

In the memory 120, one or more instructions indicating an operation to be performed by the processor 110 on data may be stored. A set of instructions may be referred to as firmware, operating system, process, routine, sub-routine and/or application. For example, the electronic device 101 and/or the processor 110 of the electronic device 101 may perform at least one of operations in FIGS. 9 to 13 by executing a set of a plurality of instructions distributed in an application form.

According to an embodiment, the communication circuit 130 of the electronic device 101 may include a hardware component for supporting transmission and/or reception of an electrical signal between the electronic device 101 and one or more external electronic devices (e.g., external electronic devices 192, 194, and 196). Although only the external electronic devices 192, 194, and 196 capable of communicating with the electronic device 101 are exemplarily illustrated, the number and/or type of external electronic devices capable of communicating with the electronic device 101 is not limited to an embodiment of FIG. 1. For example, the communication circuit 130 may include at least one of a MODEM, an antenna, and an optic/electronic (O/E) converter. The communication circuit 130 may support transmission and/or reception of the electrical signal based on various types of protocols such as Ethernet, Local Area Network (LAN), Wide Area Network (WAN), Wireless Fidelity (Wi-Fi), Bluetooth, Bluetooth Low Energy (BLE), ZigBee, Long Term Evolution (LTE), and 5G New Radio (NR).

According to an embodiment, the actuator 140 of the electronic device 101 may convert electrical energy provided to the actuator 140 into kinetic energy. For example, the actuator 140 may include a motor outputting kinetic energy by rotating a preset axis, such as a shaft. The number of the actuators 140 included in the electronic device 101 may be one or more. For example, the electronic device 101 may include actuators attachable to each of two symmetrical joints, such as hip joints. According to an embodiment, the electronic device 101 may output torque to a joint corresponding to the actuator 140 by controlling the actuator 140. For example, kinetic energy that the electronic device 101 outputs to the joint by using the actuator 140 may be outputted based on the torque.

According to an embodiment, the sensor 150 of the electronic device 101 may generate electronic information processable by the processor 110 and/or the memory 120 from non-electronic information associated with the electronic device 101. For example, the sensor 150 may include a global positioning system (GPS) sensor for detecting a geographic location of the electronic device 101.

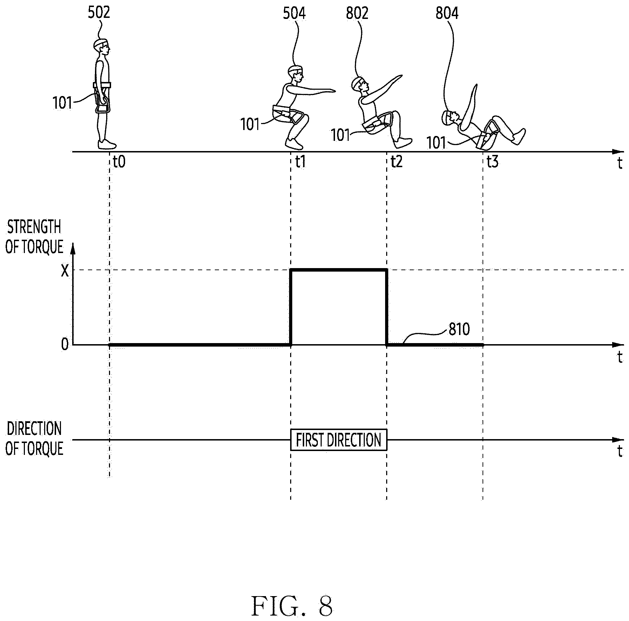

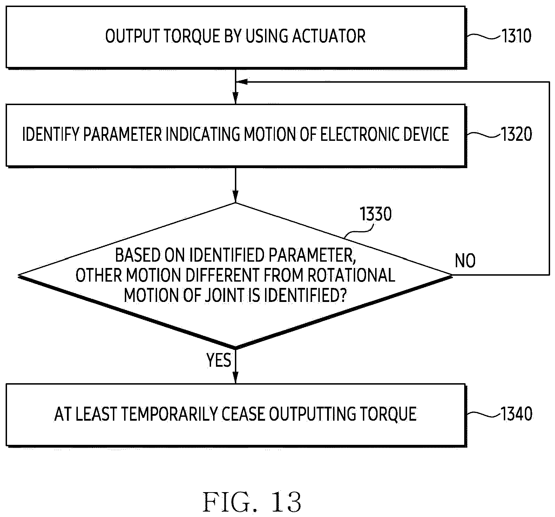

According to an embodiment, the sensor 150 of the electronic device 101 is a sensor for measuring the physical movement of the electronic device 101, and may include, for example, a gyro sensor, a gravity sensor, and/or an acceleration sensor. The gyro sensor, the gravity sensor, and the acceleration sensor may be referred to as an inertial measurement unit (IMU) sensor. For example, the gyro sensor may output information indicating an angular speed of each of a plurality of preset axes perpendicular to each other (e.g., a plurality of axes forming 90° with each other, such as x-axis, y-axis, and z-axis). For example, the acceleration sensor may output electronic information indicating magnitude of gravity acceleration measured in each of the plurality of preset axes perpendicular to each other (e.g., the x-axis, y-axis, and z-axis described above). According to an embodiment, the processor 110 of the electronic device 101 may measure motion of the electronic device 101 in a physical space based on electronic information outputted from the acceleration sensor. The motion measured by the electronic device 101 may indicate an orientation of the electronic device 101 and/or a shape of the electronic device 101 measured by the sensor 150. For example, the motion measured by the electronic device 101 may include motion of the electronic device 101 due to a fall of a user wearing the electronic device 101. An operation performed by the electronic device 101 in response to identifying the motion of the electronic device 101 due to the fall of the user will be described later with reference to FIGS. 8 and/or 13.

According to an embodiment, the electronic device 101 may output information to be provided to the user by using the speaker 160. For example, the electronic device 101 may output an acoustic signal including a preset speech to an external space of the electronic device 101 through the speaker 160. Although not shown, the electronic device 101 may output the information through a motor for outputting haptic feedback. The electronic device 101 may output the information by using one or more external electronic devices 192, 194, and 196 connected to the electronic device 101. For example, the electronic device 101 may request the external electronic devices 192, 194, and 196 including a display to display a UI corresponding to the acoustic signal through the display.

According to an embodiment, the electronic device 101 may perform controlling the user's joint using the actuator 140, based on motion of a joint identified by the sensor 150 (e.g., rotational motion generated in the joint by the user). Controlling of the joint by the electronic device 101 may be performed based on one of a plurality of preset modes. The electronic device 101 may switch between the plurality of preset modes, based on one or more parameters identified by the sensor 150 and indicating the rotational motion generated in the joint. Switching between the plurality of preset modes by the electronic device 101 will be described later with reference to FIG. 3.

According to an embodiment, while the user is performing strength exercise, the electronic device 101 may adjust the quantity of motion of the user according to the rotational motion of the joint generated by the user, by using the actuator 140. The rotational motion may cause periodic switching between a preset posture (e.g., a standby posture) and another posture in a joint corresponding to the actuator 140 of the electronic device 101. The electronic device 101 may output torque for adjusting the quantity of motion by controlling the actuator 140 in at least a part of a switching period.

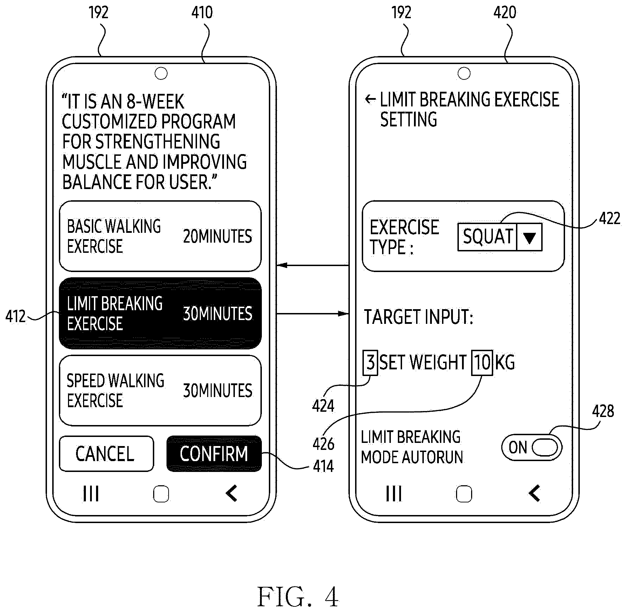

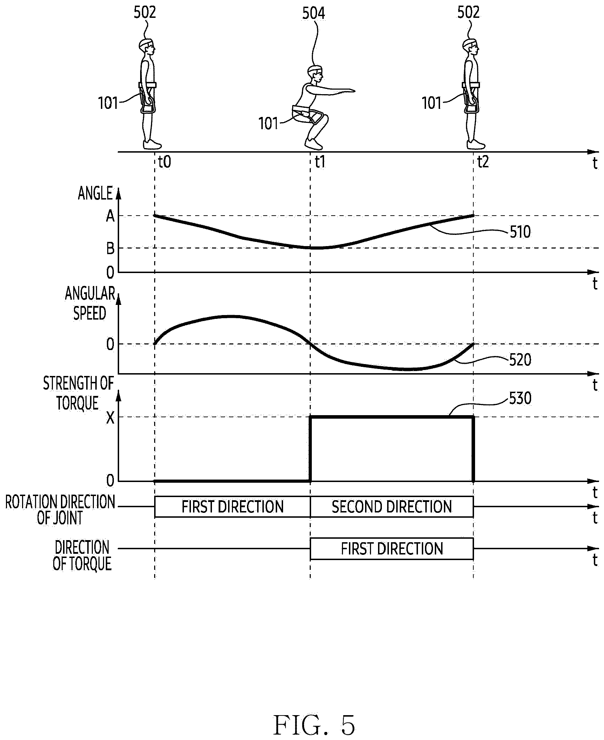

For example, the electronic device 101 may adjust strength of rotational motion, performed by the user, for converting a posture of joint corresponding to the actuator 140 into the preset posture, to preset strength, by using the actuator 140. The preset strength may be indicated by information inputted by the user. For example, the electronic device 101 may output torque for changing the strength of the rotational motion to the joint by using the actuator 140, based on the preset strength, which is inputted by the user to increase the user's quantity of motion by the rotational motion. A UI outputted by the electronic device 101 to receive the information from a user will be described later with reference to FIG. 4. An operation in which the electronic device 101 outputs the torque based on information inputted from the user will be described later with reference to FIG. 5.

According to an embodiment, the electronic device 101 may identify a parameter associated with a joint corresponding to the actuator 140 by using the sensor 150, while adjusting the strength of rotational motion based on the preset strength inputted from the user. For example, the electronic device 101 may identify one or more parameters indicating motion of the joint changed by the rotational motion. When an identified parameter satisfies a preset condition indicating a temporary cessation of the rotational motion, the electronic device 101 may change strength and/or direction of the torque outputted using the actuator 140. For example, when a parameter identified using the sensor 150 maintains a value within a preset range during a preset time section, the electronic device 101 may reduce the strength of the torque outputted using the actuator 140 or may change a direction of the torque.

According to an embodiment, adjusting the strength and/or direction of the torque outputted to the joint by the electronic device 101 may be performed to assist the user's strength exercise. As the user performs the strength exercise, a fatigue degree of one or more muscles associated with rotational motion of the joint corresponding to the actuator 140 may be increased. As the fatigue degree increases, a moment at which the strength exercise is impossible may arrive. The moment may be referred to as a critical point, a threshold point, and/or a moment of failure. According to an embodiment, the electronic device 101 may reduce strength of the torque outputted to the joint by using the actuator 140 or change a direction of the torque to strengthen the rotational motion of the j oint, in response to identifying the moment of failure using the sensor 150. In this case, the electronic device 101 may maintain performance of the strength exercise by the user even after the moment of failure. Since the performance of the strength exercise is maintained even after the moment of failure, the electronic device 101 may maximize effect of the strength exercise.

Hereinafter, a structure of the electronic device 101 according to an embodiment with a structure attachable to the user's two hip joints will be described in detail with reference to FIG. 2.

FIG. 2 is an exemplary diagram for explaining a structure of an electronic device 101, according to an embodiment. The electronic device 101 of FIG. 2 may correspond to an example of the electronic device 101 of FIG. 1. Referring to FIG. 2, the electronic device 101 may include a first support unit 201, a second support unit 202, a connection unit 203, and an actuator 140.

According to an embodiment, the electronic device 101 may be formed to be worn on a lower body of a user. Referring to FIG. 2, the first support unit 201 may be fixed to the user's waist or hip. The second support unit 202 may include a second support unit 202-1 and a second support unit 202-2. The second support unit 202 may be fixed to a leg (e.g., thigh and/or knee) of the user. For example, the second support unit 202-1 may be fixed to the left thigh of the user. The second support unit 202-2 may be fixed to the right thigh of the user.

According to an embodiment, the actuator 140 of the electronic device 101 may be coupled to the first support unit 201. The actuator 140 may include an actuator 140-1 and an actuator 140-2. The actuator 140-1 may be disposed at a first part (e.g., a left side) of the first support unit 201. The actuator 140-2 may be disposed at a second part (e.g., a right side) opposite the first part of the first support unit 201. The first part and the second part may be parts adjacent to each of the user's two hip joints in a state that the user wears the electronic device 101.

According to an embodiment, the connection unit 203 of the electronic device 101 may rotatably connect the second support unit 202 with respect to the first support unit 201 through the actuator 140. For example, the connection unit 203 may include a connection unit 203-1 and a connection unit 203-2. The connection unit 203-1 may rotatably connect the second support unit 202-1 with respect to the first support unit 201 through the actuator 140-1. For example, when the electronic device 101 outputs torque by using the actuator 140-1, rotational motion of the second support unit 202-1 centering on the first support unit 201 may occur based on strength and/or direction indicated by the torque. The connection unit 203-2 may rotatably connect the second support unit 202-2 with respect to the first support unit 201 through the actuator 140-2. For example, when the electronic device 101 outputs torque by using the actuator 140-2, a positional relationship between the first support unit 201 and the second support unit 202-2 may be changed based on the strength and/or direction indicated by the torque.

As the actuators 140-1 and 140-2 are disposed in each of the first and second parts adjacent to each of the user's two hip joints, torque of each of the actuators 140-1 and 140-2 may be outputted to each of the two hip joints. According to an embodiment, the electronic device 101 may generate torque associated with user's motion (e.g., rotational motion by strength exercise performed in at least one of the two hip joints) by using the actuator 140. For example, the torque may be generated by the actuator 140 in order to increase and/or decrease quantity of motion according to rotational motion at least one of the user's two hip joints. For example, within a time section where the user moves the hip joint to stand up, the electronic device 101 may generate first torque (e.g., torque based on a direction opposite to a direction in which the user stands up) that increases the user's quantity of motion associated with the hip joint within the time section. For another example, the electronic device 101 may generate second torque (e.g., torque based on a direction in which the user stands) to guide the user's motion within the time section. An operation in which the electronic device 101 selects torque to be generated using the actuator 140 among the first torque or the second torque may be performed based on user's motion identified by using at least one sensor (e.g., the sensor 150 of FIG. 1). For example, the electronic device 101 may change strength and/or direction of the torque to be outputted using the actuator 140, based on an angle (e.g., an angle between the user's waist and thigh) of the hip joint and/or an angular speed of the hip joint.

Although an example of the electronic device 101 having a structure attachable to two hip joints is described, the embodiment is not limited thereto. For example, the electronic device 101 may have a form-factor attachable to another joint (e.g., knee joint, shoulder joint, and/or elbow joint) different from the two hip joints. In another embodiment with a form-factor attachable to the other joint different from the two hip joints, the electronic device 101 may assist the strength exercise performed by the user in the other joint by using an actuator 140 corresponding to the other joint.

Hereinafter, an operation in which the electronic device 101 switches between a plurality of preset modes while assisting the user's strength exercise will be described with reference to FIG. 3. The plurality of preset modes may be provided to the user to overcome a threshold point, a critical point, and/or a moment of failure caused by the strength exercise.

FIG. 3 is an exemplary state transitional diagram of an electronic device, according to an embodiment. The electronic device of FIG. 3 may correspond to an example of the electronic device 101 of FIGS. 1 and/or 2.

According to an embodiment, the electronic device may operate according to a mode from among preset modes 310, 320, 330, and 340, while consuming power exceeding standby power as being activated. Switching between modes 310, 320, 330, and 340 by the electronic device may be performed based on at least one of a user input, motion of one or more joints identified by the electronic device, or motion of the electronic device. For example, switching between modes 310, 320, 330, and 340 may be performed to assist motion (e.g., motion associated with the strength exercise) performed by the user wearing the electronic device.

Referring to FIG. 3, the mode 310 may be referred to as a manual mode. In the mode 310, the electronic device may deactivate one or more actuators (e.g., the actuator 140 of FIGS. 1 to 2) included in the electronic device. Deactivating the actuator by the electronic device may include an operation of controlling the actuator independently of changing movement of a joint corresponding to the actuator. For example, in the mode 310, the user of the electronic device may move a joint corresponding to an actuator independently of the actuator. In the mode 310, the electronic device may cease to input power to the actuator at least temporarily.

Referring to FIG. 3, the mode 320 may be referred to as a resistance mode. In the mode 320, the electronic device may activate one or more actuators included in the electronic device. By controlling one or more activated actuators, the electronic device may output torque based on a first direction and having a first strength to one or more joints corresponding to the one or more actuators. The first direction may correspond to a direction different from a direction of rotational motion generated by the user at one or more joints. For example, the first strength may be information inputted by the user of the electronic device, and may correspond to a target weight. While the electronic device operates based on the mode 320, the user wearing the electronic device may perform rotational motion according to quantity of motion increased by the torque outputted from the actuator. For example, the user may perform the rotational motion based on the target weight. In mode 320, the electronic device may control the actuator so that the quantity of motion of the user performing strength exercise is increased based on the target weight indicated by the information.

Referring to FIG. 3, the mode 330 may be referred to as a first assist mode and/or a soft-assist mode. In the mode 330, the electronic device may activate one or more actuators. By controlling one or more activated actuators, the electronic device may output torque based on the first direction of the mode 320 and having second strength different from the first strength of the mode 320 to one or more joints corresponding to the one or more actuators. For example, the second strength may be strength less than the first strength, and may correspond to a weight less than the target weight. While the electronic device operates based on the mode 330, the user wearing the electronic device may perform rotational motion, according to quantity of motion, which is outputted from the actuator and is less than the quantity of motion corresponding the target weight of the mode 320. For example, in mode 330, the electronic device may control the actuator so that the quantity of motion of the user performing strength exercise is increased, based on the weight less than the target weight.

Referring to FIG. 3, in modes 320 and 330, as the electronic device outputs torque based on a first direction corresponding to a direction different from the direction of the rotational motion generated by the user, the user may experience motion of the electronic device resisting the rotational motion performed by the user. For example, in the mode 320, the user may experience motion of the electronic device resisting the user's the rotational motion based on the target weight. For example, in the mode 330, the user may experience the motion of the electronic device resisting the user's rotational motion based on a weight less than the target weight.

Referring to FIG. 3, the mode 340 may be referred to as a second assist mode, a hard-assist mode, and/or a strong-assist mode. In the mode 340, the electronic device may activate one or more actuators. By controlling one or more activated actuators, the electronic device may output torque of third strength, which is independent of the first strength and the second strength of the modes 320 and 330 and based on a second direction different from the first direction of the modes 320 and 330, to one or more joints corresponding to one or more actuators. The second direction may correspond to a direction of rotational motion generated by the user at one or more joints. In mode 340, according to an embodiment, the electronic device may output torque based on the second direction and having the third strength in order to compel the rotational motion of the joint corresponding to the actuator. For example, in response to switching from modes 320 and 330 that output torque based on the first direction opposite to the direction of the rotational motion to mode 340, the electronic device may output torque based on the second direction matching the direction of the rotational motion. In the mode 340, as the electronic device outputs torque based on the second direction matching the rotational motion generated by the user, the user may experience motion of the electronic device assisting rotational motion performed by the user. For example, the user may experience motion of the electronic device that ease the rotational motion based on a negative weight.

In at least one of the modes 330 and 340, according to an embodiment, the electronic device may output a preset message associated with the strength exercise. For example, the preset message may include a preset comment (e.g., comment to guide the performance of strength exercise, such as "Do it one more time") based on an acoustic signal format. For example, the preset message may include preset text (e.g., text corresponding to the comment) displayed on a display (e.g., at least one display of the external electronic devices 192, 194, and 194 in FIG. 1) of an external electronic device connected to the electronic device.

In the modes 320, 330, and 340, outputting the torque by the electronic device may be performed in at least a part of a time section operating based on each of the modes 320, 330, and 340. As the user performs strength exercise, the user's posture may be repeatedly changed between a preset posture such as a standby posture and another posture different from the preset posture. According to an embodiment, the electronic device may output torque based on one of the modes 320, 330, and 340, while changing from the other posture to the preset posture. For example, the electronic device may at least temporarily cease to output torque based on one of the modes 320, 330, and 340, while changing from the preset posture to the other posture. An operation in which the electronic device outputs torque according to a change in the user's posture in each of the modes 320, 330, and 340 will be described later with reference to FIGS. 5 to 7.

According to an embodiment, switching between the modes 310, 320, 330, and 340 of FIG. 3 by the electronic device may be performed by at least one of a user input, user's motion identified in the electronic device, and motion of the electronic device. Hereinafter, an operation in which the electronic device switches between the modes 310, 320, 330, and 340 described above will be described.

In the mode 310, according to an embodiment, the electronic device may be switched to the mode 320 along a direction 352, in response to identifying initiation of the rotational motion by the user. For example, switching from the mode 310 to the mode 320 along the direction 352 may be performed based on information obtained from the user by using an external electronic device (e.g., the external electronic device 192 of FIG. 1) different from the electronic device. For example, the electronic device may identify initiation of the rotational motion, based on one or more parameters identified using at least one sensor (e.g., the sensor 150 of FIG. 1) included in the electronic device.

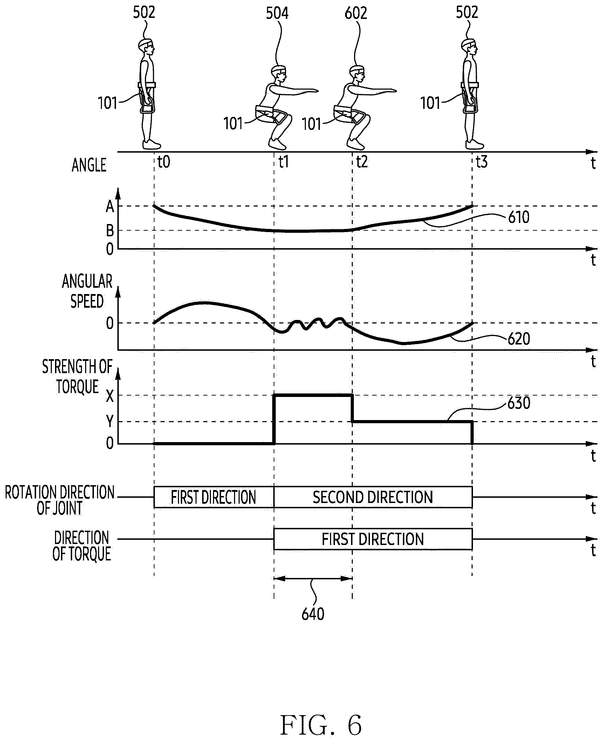

According to an embodiment, switching the electronic device from the mode 320 to the mode 330 along a direction 354 may be performed based on rotational motion of one or more joints identified by the electronic device using at least one sensor (e.g., the sensor 150 of FIG. 1). For example, in response to identifying that the user's rotational motion is ceased or slowed, the electronic device may be switched from the mode 320 to the mode 330. For example, switching from the mode 320 to the mode 330 along the direction 354 by the electronic device may be performed, in response to identifying that one or more parameters identified using at least one sensor satisfy a preset condition associated with a moment of failure. An example of the preset condition will be described later with reference to FIG. 6. For example, in response to identifying that the user reaches the moment of failure, the electronic device may reduce strength of torque outputted to a joint by switching to the mode 330. Based on the torque having the reduced strength, the electronic device may cause the user to overcome a limit of strength exercise.

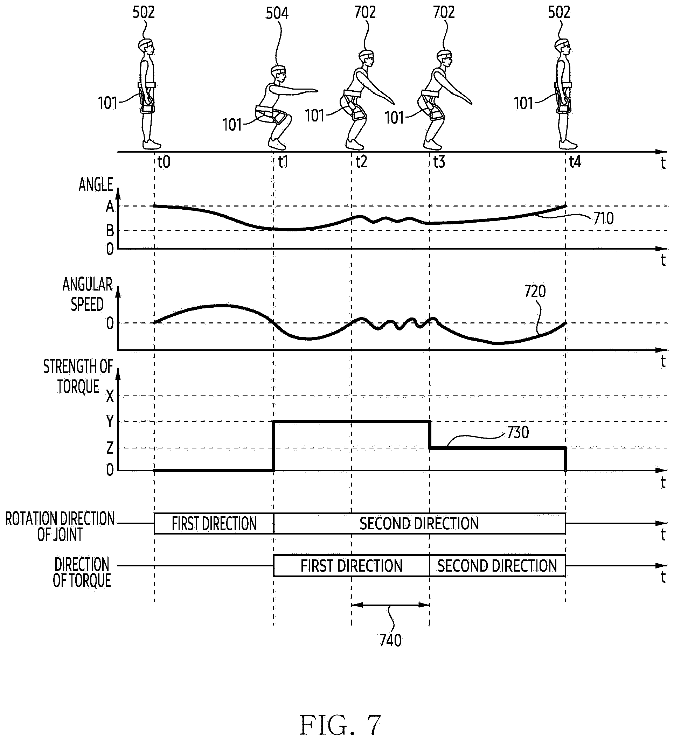

According to an embodiment, switching to the mode 340 along directions 356 and 362 by the electronic device may be performed based on one or more parameters identified by the electronic device using at least one sensor and indicating rotational motions of one or more joints. For example, in response to identifying that the user satisfies a preset condition associated with moment of failure after switching to the mode 330 or identifying that another preset condition for bypassing the mode 330 and switching to the mode 340 is satisfied in the mode 320, the electronic device may switch to the mode 340. An example of a condition for switching to the mode 340 will be described later with reference to FIG. 7. According to switching to the mode 340 from a mode among the modes 320 and 330, the electronic device may ease the user to complete the rotational motion.

According to an embodiment, switching from the mode 340 to mode the 310 along the direction 358 by the electronic device may be performed in response to receiving a preset user input to cease assistance of strength exercise by the electronic device. For example, in the mode 340, the electronic device may output a message for verifying whether to cease the strength exercise based on an acoustic signal format through a speaker (e.g., the speaker 160 of FIG. 1) or display the message by using one or more visual objects on a display (e.g., a display of at least one of the external electronic devices 192, 194, and 196 of FIG. 1) of an external electronic device. In response to receiving a user input corresponding to the message, the electronic device may switch from the mode 340 to the mode 310. According to switching to the mode 310, the electronic device may at least temporarily cease to assist the user's strength exercise.

Referring to FIG. 3, the electronic device may switch to the mode 310 along the directions 372, 374, and 376, in order to cease assisting the strength exercise, independently of a preset user input to cease assisting the strength exercise. For example, switching to the mode 310 from other modes 320, 330, and 340 different from the mode 310 may be performed in response to identifying that a preset condition associated with an emergency stop is satisfied. For example, when a user wearing the electronic device falls, and/or a switch (e.g., a button associated with the emergency stop) exposed to the outside through a housing of the electronic device is pressed, the electronic device may cease to output the torque, based on at least one of the modes 320, 330, and 340, by switching to the mode 310. An operation in which the electronic device performs based on the preset condition associated with the emergency stop will be described later with reference to FIG. 8.

As described above, according to an embodiment, the electronic device may assist the user's strength exercise based on the modes 310, 320, 330, and 340. For example, in the modes 320 and 330, the electronic device may control one or more actuators to increase the user's quantity of motion. For example, in the mode 340, the electronic device may control one or more actuators so that the performance of the rotational motion by the user is maintained. As the modes 320, 330, and 340 are sequentially switched, the user wearing the electronic device may additionally perform strength exercise at a moment after a critical point. As the user additionally performs strength exercise after the critical point, the effect of strength exercise may be increased.

Hereinafter, according to an embodiment, an operation in which the electronic device receives information associated with the user's strength exercise by using an external electronic device will be described with reference to FIG. 4.

FIG. 4 is a diagram illustrating an example of a user interface (UI) displayed by an external electronic device 192 associated with an electronic device, according to an embodiment. The external electronic device 192 of FIG. 4 may correspond to an example of the external electronic device 192 of FIG. 1. The electronic device of FIG. 4 may correspond to an example of the electronic device 101 of FIG. 1.

Referring to FIG. 4, according to an embodiment, in a state that a connection such as pairing is established between the electronic device and the external electronic device 192, screens 410 and 420 provided by the electronic device to the user through a display of the external electronic device 192 are illustrated. Hereinafter, the screen may mean a user interface (UI) displayed in at least a part of the display. For example, the screen may include Activity of an Android operating system.

Referring to FIG. 4, the external electronic device 192 may display screens 410 and 420 for receiving one or more parameters associated with driving of the electronic device. In the screen 410, the external electronic device 192 may display a plurality of parameters adjustable by a user wearing the electronic device by using one or more visual objects, according to the type of motion associated with each of the plurality of parameters. For example, a button 412 in the screen 410 may cause switching to the screen 420 for receiving one or more parameters associated with the user's strength exercise. When the user clicks and/or touches the button 412, the external electronic device 192 may switch from the screen 410 to the screen 420.

Referring to FIG. 4, in the screen 420, the external electronic device 192 may display one or more visual objects for obtaining at least one parameter associated with the strength exercise. For example, by using a visual object such as a combo box 422, the external electronic device 192 may receive a user input for selecting the type of the strength exercise. For example, by using visual objects such as text boxes 424 and 426, the external electronic device 192 may receive information indicating a target weight and/or a target number. For example, by using a visual object such as a toggle button 428, the external electronic device 192 may receive a user input for activating a mode (e.g., modes 320, 330, and 340 the FIG. 3) that outputs torque associated with the strength exercise using the electronic device.

According to an embodiment, a parameter received by the external electronic device 192 through the screen 420 is not limited to an example of FIG. 4. For example, the external electronic device 192 may receive information necessary to identify a failure moment associated with the type of the strength exercise indicated by the combo box 422. For example, the information may include at least one of a threshold of a heart rate, an angle of a hip joint, or a threshold of a peak value of an angular speed.

In an example of FIG. 4, the external electronic device 192 may transmit one or more parameters (e.g., a value indicated by text boxes 424 and 426 and/or a combo box 422) identified through the screen 420 to the electronic device, in response to identifying a user input that allows switching to a mode to assist the strength exercise by the toggle button 428. Alternatively, the external electronic device 192 may transmit the one or more parameters to the electronic device, in response to receiving a user input associated with a visual object, such as a button 414, for completing an input of a parameter based on screens 410 and 420. After transmitting the one or more parameters, when the user wearing the electronic device performs strength exercise (e.g., squat exercise indicated by the combo box 422), the electronic device may operate according to a preset mode (e.g., one of the modes 320, 330, and 340 of FIG. 3) for assisting the strength exercise.

For example, based on the mode 320 of FIG. 3, the electronic device may output torque corresponding to a target weight (e.g., 10kg) indicated by the text box 426 to the user's hip joint using the actuator. The electronic device may change strength and/or direction of the torque outputted to the hip joint based on at least one of the modes 330 and 340 of FIG. 3, in response to identifying that a target number indicated by the text box 424 (e.g., a number corresponding to one set) has been reached, or identifying a state of a user who has reached a failure point.

Hereinafter, an operations performed by the electronic device in a state that switching to a mode for assisting the strength exercise based on a toggle button 428 is allowed by the user will be described in detail with reference to FIGS. 5 to 7.

FIG. 5 is an exemplary diagram for illustrating an operation performed by an electronic device 101 according to a user's motion, according to an embodiment. The electronic device 101 of FIG. 5 may correspond to an example of the electronic device 101 of FIGS. 1 to 2 and/or the electronic device of FIGS. 3 to 4. Hereinafter, it is assumed that the electronic device 101 of FIG. 5 operates based on the mode 320 among the modes 310, 320, 330, and 340 of FIG. 3.

Referring to FIG. 5, in a state that a user wearing the electronic device 101 repeatedly moves between postures 502 and 504, one or more parameters associated with a joint detected by the electronic device 101 and the strength and direction of torque outputted by the electronic device 101 using an actuator are illustrated. For example, a posture 502 may correspond to a posture in which quantity of motion required to maintain the posture is minimized, among a plurality of postures associated with the strength exercise. Hereinafter, the posture 502 may be referred to as a standby posture and/or a preset posture. For example, a posture 504 may correspond to a posture in which the quantity of motion required to maintain the posture is maximized, among the plurality of postures associated with the strength exercise.

Referring to FIG. 5, as the user starts the strength exercise from a moment t0, the user's posture may change from the posture 502 to the posture 504 between the moment t0 and a moment 11. A direction of rotational motion generated by the user between the moment t0 and the moment t1 may correspond to a first direction reducing an angle between waist and thigh from an angle A (e.g., 180°) to an angle B (e.g., 90°) less than the angle A. As the user stands up after the moment t1, a user's posture may be restored from the posture 504 to the posture 502. A direction of rotational motion generated by the user between the moment t1 and a moment t2 may correspond to a direction in which an angle between the waist and the thigh increases from the angle B to the angle A, such as a second direction opposite to the first direction.

Referring to FIG. 5, graphs 510 and 520 corresponding to each of a plurality of parameters detected by the electronic device 101 using at least one sensor (e.g., the sensor 150 of FIG. 1) and associated with the strength exercise between the moment t0 and the moment t2 when the user performs the strength exercise are illustrated. The graph 510 indicates an angle of a user's joint (at least one of the two hip joints in an example of FIG. 5) detected by electronic device 101. The graph 520 indicates an angular speed of the joint detected by electronic device 101. A type of parameter detected by the electronic device 101 is not limited to an example of FIG. 5. For example, in case that the electronic device 101 is connected to an external electronic device (e.g., the external electronic device 194 of FIG. 1), between the moment t0 and the moment t2, the electronic device 101 may additionally obtain another parameter different from parameters detectable using a sensor of the electronic device 101, such as a heart rate, from the external electronic device.

Referring to the graph 510, between the moment t0 and the moment t2, an angle of the joint detected by the electronic device 101 may have a maximum value (e.g., an angle A corresponding to 180°) at the moments t0 and t2 corresponding to the posture 502, and a minimum value (e.g., an angle B corresponding to 90°) at the moment t1 corresponding to the posture 504. Referring to the graph 520, between the moment t0 and the moment t2, magnitude of the angular speed of the joint detected by the electronic device 101 may substantially correspond to zero in moments t0, t1, and t2 in which a rotation direction of the joint is changed.

According to an embodiment, the electronic device 101 may output torque using the actuator, in response to identifying rotational motion, which is performed by the user based on a joint corresponding to the actuator of the electronic device 101, for restoring the user's posture to a standby posture (e.g., the posture 502). Referring to FIG. 5, between the moment t1 and the moment t2, the user wearing the electronic device 101 may perform rotational motion for restoring from the posture 504 to the posture 502 by rotating the hip joint along the second direction. According to an embodiment, the electronic device 101 may identify the rotational motion for rotating the hip joint along the second direction based on at least one of the angle of the hip joint indicated by the graph 510 and/or the angular speed of the hip joint indicated by the graph 520.

The electronic device 101 may initiate outputting torque by controlling the actuator, in response to identifying the rotational motion based on the second direction. Referring to FIG. 5, strength of the torque outputted by the electronic device 101 to the hip joint using the actuator is illustrated based on the graph 530. For example, the electronic device 101 may output torque having strength X corresponding to information (e.g., a target weight indicated by the text box 426 of FIG. 4) identified by the user and based on another direction (e.g., the first direction) different from a rotation direction of the joint, based on the mode 320 of FIG. 3. Outputting the torque by the electronic device 101 may be performed while the rotational motion based on the second direction is performed by the user. Referring to the graph 530 of FIG. 5, between the moment t1 and the moment t2, the electronic device 101 may output the torque based on the first direction and having the strength X, to the hip j oint using an actuator. Outputting the torque by the electronic device 101 may be maintained until to the moment t2 in which the user's posture is restored to the posture 502 corresponding to the standby posture. Between the moment t1 and the moment t2, the electronic device 101 may increase the user's quantity of motion based on the torque indicated by the graph 530.

As the user repeatedly performs the strength exercise after the moment t2, the electronic device 101 may repeatedly identify an angle and acceleration changing based on each of the graphs 510 and 520 between the moment t0 and the moment t2 of FIG. 5. While repeatedly identifying the angle and the acceleration, the electronic device 101 may output torque resisting to the user's rotational motion restoring to the posture 502 using the actuator, similar as indicated by the graph 530. The torque may increase the quantity of motion consumed by the user to restore to the posture 502.

As described above, according to an embodiment, the electronic device 101 may increase the user's quantity of motion by outputting torque assisting the user's strength exercise. As the user repeatedly performs the strength exercise, fatigue of the user's muscles (e.g., muscles associated with the hip joint exercise) may be increased. As the fatigue of the muscles increases, the angle and/or acceleration indicated by the graphs 510 and 520 may change differently. For example, when the user reaches the limit of performing the strength exercise as the fatigue increases, the angle and/or acceleration measured by the electronic device 101 may be changed differently from shapes of the graphs 510 and 520.

Hereinafter, an operation performed by the electronic device 101 in a state that the user reaches the limit will be described in detail with reference to FIG. 6.

FIG. 6 is an exemplary diagram for illustrating another operation performed by an electronic device 101 according to a user's motion, according to an embodiment. The electronic device 101 of FIG. 6 may correspond to an example of the electronic device 101 of FIGS. 1, 2, and/or 5 and/or the electronic device of FIGS. 3 to 4. For example, an operation of the electronic device 101 of FIG. 6 may correspond to an example of switching to the mode 330 in a state of operating based on the mode 320 among the modes 310, 320, 330, and 340 of FIG. 3.

Referring to FIG. 6, an exemplary state in which a user wearing the electronic device 101 has reached a limit of performing strength exercise is illustrated. For example, a moment t0 to a moment t3 in FIG. 6 are moments after the moment t0 to the moment t2 in FIG. 5, and may correspond to a moment after the user repeatedly switches a posture between postures 502 and 504 in FIG. 5. For example, according to an embodiment, as the user moves according to rotational motion based on a second direction for restoring to the posture 502 corresponding to a standby posture after the moment t1, the electronic device 101 may output torque based on a first direction different from the second direction and having strength X based on information inputted from the user. For example, the strength X may be associated with a target weight inputted by the user. Outputting the torque by the electronic device 101 may be performed based on, for example, the mode 320 of FIG. 3.

According to an embodiment, the electronic device 101 may identify an angle and angular speed of a joint (e.g., hip joint) associated with the electronic device 101, after being activated. Graphs 610 and 620 of FIG. 6 may correspond to each of the angle and the angular speed identified by the electronic device 101. According to an embodiment, a parameter identified by the electronic device 101 is not limited to the angle and the angular speed, and the electronic device 101 may include, for example, a parameter indicating motion of the electronic device 101 indicated by the IMU sensor and/or a parameter (e.g., a parameter indicating the user's heart rate) received from an external electronic device (e.g., one or more external electronic devices 192, 194, and 196 in FIG. 1) connected to the electronic device 101.

As the user performs strength exercise, fatigue may be increased. As the user's fatigue increases, a range in which the angle of the joint is changed, identified by the electronic device 101 and indicated by the graph 610 may be reduced. As the user's fatigue increases, a maximum value of the angular speed of the joint identified by the electronic device 101 and indicated by the graph 620 may gradually decrease. When the electronic device 101 receives the user's heart rate using an external electronic device, the heart rate may increase as the user performs the strength exercise.

Referring to FIG. 6, an exemplary state in which the user reaches a limit of performing the strength exercise after the moment t1 is illustrated. For example, the user may maintain the posture 504 of the moment t1 during a preset time section 640 after the moment 11. For example, at the moment t2 after the moment t1, the user may maintain a posture 602 similarly to the posture 504. According to an embodiment, the electronic device 101 may identify that it is difficult for the user to restore to the posture 502 corresponding to the standby posture, based on at least one of the angle of the j oint indicated by the graphs 610 and 620, the angular speed, or the user's heart rate received from the external electronic device. For example, the electronic device 101 may identify that it is difficult for the user to restore to the posture 502 based on conditions in Table 1.

**[Table 1]**

| Angle (A) | Angular speed (B) | Heart rate (C) |
|---|---|---|
| Condition (A1) | Condition (B1) | Condition (C1) |
| An angle is included within a preset range different from an angle corresponding to the posture 502, for a preset time section (e.g., N seconds). | An absolute value of an angular speed is included within a preset range, for a preset time section (e.g., N seconds). | A heart rate increases above a preset increase rate, for a preset time section (e.g., N seconds). |
| Condition (A2) | Condition (B2) | Condition (C2) |
| An angle is maintained as an angle different from the angle corresponding to the posture 502, for a preset time section (e.g., N seconds). | An absolute value of an angular speed is maintained below a preset threshold, for a preset time section (e.g., N seconds). | A heart rate exceeds a preset threshold, for a preset time section (e.g., N seconds). |

Referring to Table 1, conditions A1 and A2 associated with the angle may indicate that an angle of a user's joint is maintained for a long time at an angle corresponding to another posture different from the posture 502 corresponding to the standby posture. Referring to Table 1, conditions B1 and B2 associated with the angular speed may indicate that the user's motion to restore to the posture 502 corresponding to the standby posture is at least temporarily ceased. Referring to Table 1, conditions C1 and C2 associated with the heart rate may indicate a rapid increase in the user's heart rate. A length (e.g., N seconds) of each of the conditions A1, A2, B1, B2, C1, and C2, a preset range, and/or a preset threshold may be changed experientially based on log data collected from one or more users including the user of the electronic device 101. According to an embodiment, the electronic device 101 may reduce strength of torque being outputted using an actuator based on whether each of the angle, angular speed, and heart rate satisfies the conditions of Table 1. For example, the electronic device 101 may change the strength of the torque from strength X to strength Y less than the strength X, in response to identifying that at least one of the conditions A1 and A2 associated with the angle and at least one of the conditions B1 and B2 associated with the angular speed are satisfied at the same time. For example, when at least one of the conditions C1 and C2 associated with the heart rate is satisfied, the electronic device 101 may reduce the strength of the torque, in response to identifying that at least one of the conditions A1, A2, B1, and B2 associated with the angle and/or angular speed is satisfied.

In an exemplary situation of FIG. 6, in the time section 640 between the moment t1 and the moment t2, as an angle indicated by the graph 610 is maintained as an angle different from the angle corresponding to the posture 502, the electronic device 101 may identify that the condition A2 of Table 1 is satisfied. In the time section 640, as an absolute value of an angular speed indicated by the graph 620 is maintained below a preset threshold, the electronic device 101 may identify that the condition B2 of Table 1 is satisfied. In the time section 640, in response to identifying that all of the condition A2 associated with the angle and the condition B2 associated with the angular speed are satisfied, the electronic device 101 may change the strength of the torque to the strength Y less than the strength X. For example, the electronic device 101 may switch from the mode 320 of FIG. 3 to the mode 330.

Referring to FIG. 6, in a state that the electronic device 101 switches from the mode 320 of FIG. 3 to the mode 330, the strength of the torque outputted by the electronic device 101 using the actuator is illustrated as the graph 630. Referring to the graph 630, the electronic device 101 may output torque having the strength Y corresponding to a weight less than a target weight. The torque may be outputted to the user's joint, based on a first direction different from a second direction, which is a direction of rotational motion by the user. For example, after the moment t2, the electronic device 101 may operate based on the mode 330 of FIG. 3. Changing the strength of the torque at the moment t2 by the electronic device 101 may be associated with switching to the mode 330 along the direction 354 of FIG. 3.

According to an embodiment, after the moment t2, the electronic device 101 may output torque resisting the user's rotational motion restoring to the posture 502 based on strength less than the strength X corresponding to the target weight (in an example of FIG. 6, the strength Y), based on the mode 330 of FIG. 3. While outputting the torque, the electronic device 101 may output a preset acoustic signal (e.g., an acoustic signal including a comment such as, "You can do it! Cheer up a little bit more! ") to guide restoring to the posture 502.

Referring to FIG. 6, after the moment t2, the strength of the torque outputted by the electronic device 101 may be reduced to resist the user's rotational motion restoring to the posture 502. As the strength of the torque is reduced, the user may more easily complete switching to the posture 502 corresponding to the standby posture. For example, at the moment t3 after the moment t2, the user's posture may correspond to the posture 502. After the moment t3, the user may repeatedly perform the strength exercise, based on the strength Y corresponding to a weight less than the target weight. As the electronic device 101 adjusts torque outputted using the actuator based on a preset condition (e.g., at least one of conditions in Table 1) as shown in graph 630, even though the threshold is reached, the user may continuously perform the strength exercise. According to an embodiment, the electronic device 101 may gradually reduce the strength of the torque outputted using the actuator so that the user may continuously perform the strength exercise after the moment t3. According to an embodiment, the electronic device 101 may maintain performing continuously the strength exercise by the user, based on, for example, the number of repetitions inputted through the text box 424 of FIG. 4.

Hereinafter, an operation in which the electronic device 101 performs in response to identifying that the user reaches a limit of the strength exercise after adjusting the strength of the torque outputted using the actuator to the strength corresponding to a weight less than the target weight will be described in detail with reference to FIG. 7.

FIG. 7 is an exemplary diagram for illustrating still another operation performed by an electronic device 101 according to a user's motion, according to an embodiment. The electronic device 101 of FIG. 7 may correspond to an example of the electronic device 101 of FIGS. 1, 2, 5 and/or 6 and/or the electronic device of FIGS. 3 to 4. For example, an operation of the electronic device 101 of FIG. 7 may correspond to an example of switching to the mode 340 in a state of operating based on the mode 330 among the modes 310, 320, 330, and 340 of FIG. 3.

A moment t0 to a moment t4 in FIG. 7, which are moments after the moment t0 to the moment t2 in FIG. 5 and/or the moment t0 to the moment t3 in FIG. 6, may correspond to a moment after the user repeatedly switches a posture between the postures 502 and 504 in FIG. 5 and/or 6. For example, before the moment t3, the electronic device 101 may output torque having strength Y corresponding to a weight less than a target weight and based on a first direction different from a second direction, which is a direction of rotational motion of a joint for restoring to the posture 502, by using the actuator based on the mode 330 of FIG. 3.

Referring to FIG. 7, graphs 710 and 720 indicating each of an angle and angular speed of a j oint detected using a sensor by the electronic device 101 are illustrated. In a state of outputting torque based on the mode 330 of FIG. 3, the electronic device 101 may identify that restoring to the posture 502 is difficult as the user reaches the limit of the strength exercise, based on at least one of the angle or the angular speed. For example, the electronic device 101 may identify that it is difficult to restore to the posture 502 by the user based on conditions in Table 2.

**[Table 2]**

| Angle (A) | Angular speed (B) |
|---|---|
| Condition (A3) | Condition (B3) |
| An angle is included within a preset range different from an angle corresponding to the posture 502, for a preset time section (e.g., N seconds). | An angular speed is included within a preset range for a preset time section (e.g., N seconds). |

Referring to Table 2, the condition A3 associated with the angle may indicate that an angle of a user's joint is maintained for a long time at an angle corresponding to another posture different from the posture 502 corresponding to the standby posture. Referring to Table 1, the condition B3 associated with the angular speed may indicate that the user's motion for restoring to the posture 502 corresponding to the standby posture is at least temporarily ceased or that motion opposite to the motion may be generated. A length of a time section of each of the conditions A3 and B3 and/or preset range may be determined independently of the conditions A1, A2, B1, and B2 of Table 1. A length of a time section of each of the conditions A3 and B3 and/or preset range may be changed experientially based on log data collected from one or more users including the user of the electronic device 101. According to an embodiment, the electronic device 101 may change a direction of torque being outputted using the actuator, based on whether each the angle and the angular speed detected using at least one sensor and indicated by each of the graphs 710 and 720 satisfies the conditions in Table 2. For example, in response to identifying that at least one of the condition A3 associated with the angle and the condition B3 associated with the angular speed is satisfied, the electronic device 101 may change a direction of the torque to a direction, which is based on the second direction for restoring to the posture 502. Changing the direction of the torque by the electronic device 101 may be performed by switching to the mode 340 of FIG. 3. For example, changing the direction of the torque by the electronic device 101 may be associated with switching to the mode 340 along at least one of the directions 356 and 362 of FIG. 3.

In an exemplary situation of FIG. 7, as the user maintains a posture 702 different from the postures 502 and 504 within a time section 740 between the moment t2 to the moment t3, the electronic device 101 may identify that the angle and angular speed indicated by the graphs 710 and 720 satisfy the conditions A3 and B3 of Table 2. In response to identifying that the conditions A3 and B3 of Table 2 are satisfied within the time section 740, after the moment t3, the electronic device 101 may change strength of the torque outputted using the actuator to preset strength Z independent of the strength X and the strength Y as shown in the graph 730. After the moment t3, the electronic device 101 may change a direction of the torque outputted using the actuator to a direction, which is based on the second direction.

Referring to FIG. 7, after the moment t3, as the electronic device 101 outputs torque having the strength Z and a direction corresponding to the second direction, which is a direction of rotational motion for restoring to the posture 502, by using the actuator, the user may change the user's posture more easily to the posture 502 corresponding to the standby posture. For example, as the electronic device 101 outputs the torque, the user may quickly escape from a state in which the posture 702 is maintained. For example, even though a limit is reached in the time section 740, the user may switch to the posture 502 by using the torque outputted from electronic device 101. According to an embodiment, the electronic device 101 may maintain outputting torque having a direction, which is based on the second direction, using an actuator so that the user may continuously perform the strength exercise after the moment t4. For example, the electronic device 101 may maintain outputting the torque based on the number of repetitions inputted through the text box 424 of FIG. 4.

As described above based on FIGS. 5 to 7, according to an embodiment, the electronic device 101 may output torque having a direction and strength for assisting the user's strength exercise. The electronic device 101 may change the direction and/or strength of the torque based on a user's state associated with the strength exercise. For example, the electronic device 101 may identify the angle and/or angular speed of the user's j oint, by using at least one sensor. For example, the electronic device 101 may identify the user's heart rate from an external electronic device. The angle, the angular speed, and the heart rate may be used to identify the user's state associated with the strength exercise by the electronic device 101. For example, the electronic device 101 may guide the user's rotational motion associated with the strength exercise based on quantity of motion increased or decreased based on the user's condition. Guiding the rotational motion by the electronic device 101 may be performed to overcome a state in which the strength exercise is impossible to perform, such as a failure moment.

Hereinafter, according to an embodiment, an operation in which the electronic device 101 performs an emergency stop will be described in detail with reference to FIG. 8.

FIG. 8 is an exemplary diagram for illustrating an operation in which an electronic device 101 cease to output torque by using an actuator, according to an embodiment. The electronic device 101 of FIG. 8 may correspond to an example of the electronic device 101 of FIGS. 1 to 2, the electronic device of FIGS. 3 to 4, and/or the electronic device 101 of FIGS. 5 to 7. For example, an operation of the electronic device 101 of FIG. 8 may correspond to an example of switching from one of the modes 320, 330, and 340 of FIG. 3 to the mode 310.

Referring to FIG. 8, an exemplary situation in which a user wearing the electronic device 101 falls while performing the strength exercise that requires repeatedly switching between the postures 502 and 504 the is illustrated. Between the moment t0 and the moment t1, the user may switch from the posture 502 corresponding to the standby posture to the posture 504. After the moment t1, the user may perform rotational motion for switching from the posture 504 to the posture 502. Referring to FIG. 8, at the moment t2 after the moment t1, as shown in the posture 802, the user may fall according to losing balance. At the moment t3 after the moment t2, the user may collide with a floor as shown in the posture 804.

According to an embodiment, after being activated, the electronic device 101 may detect motion of the electronic device 101 by using at least one sensor (e.g., the sensor 150 of FIG. 1). For example, the electronic device 101 may identify a direction and/or magnitude of gravitational acceleration based on preset axes. The electronic device 101 may detect a change in the gravitational acceleration according to a fall of the user wearing the electronic device 101 by using at least one sensor. For example, when gravitational acceleration detected using a sensor of the electronic device 101 such as the IMU sensor is included within a preset range indicating a state in which the user falls, the electronic device 101 may cease to output torque to the user's joint using at least one actuator (e.g., the actuator 140 of FIG. 1).

For example, referring to FIG. 8, after the moment t2, in response to detecting that the user falls, the electronic device 101 may change the strength of the torque being outputted using the actuator to 0 substantially, as shown in the graph 810 indicating the strength of the torque. As the electronic device 101 ceases to change the user's quantity of motion and/or rotational motion by using the actuator after the moment t2, the user may be prevented from being injured according to a change in the rotational motion by the actuator. Adjusting the strength of the torque being outputted using the actuator after the moment t2 by the electronic device 101 may be associated with switching to the mode 310 along the directions 372, 374, and 376 of FIG. 3.

As described above, according to an embodiment, the electronic device 101 may continuously identify whether the user falls at one or more modes (e.g., the modes 320, 330, and 340 of FIG. 3) that output torque by using the actuator. In response to identifying that the user falls, the electronic device 101 may cease to output the torque using the actuator.

Hereinafter, according to an embodiment, an operation performed by the electronic device 101 will be described in detail with reference to FIGS. 9 to 13.

FIG. 9 is a flowchart illustrating an operation performed by an electronic device according to an embodiment. The electronic device of FIG. 9 may correspond to an example of the electronic device 101 of FIGS. 1 to 2, 5 to 8, and/or the electronic device of FIGS. 3 to 4. For example, at least one of operations of FIG. 9 may be performed by the electronic device 101 of FIG. 1 and/or the processor 110 of FIG. 1.

Referring to FIG. 9, according to an embodiment, in operation 910, the electronic device may identify rotational motion of a user's joint. For example, when a user wearing the electronic device initiates the strength exercise, the electronic device may identify that the user has initiated the strength exercise by using at least one sensor (e.g., the sensor 150 of FIG. 1). As the user performs the strength exercise, the electronic device may identify that a posture of the user's joint corresponding to an actuator (e.g., the actuator 140 of FIG. 1) is repeatedly switched between a standby posture (e.g., the posture 502 of FIGS. 5 to 8) and another posture (e.g., the posture 504 of FIGS. 5 to 8) different from the standby posture. In response to identifying rotational motion of the user's joint, the electronic device may operate based on the mode 320 of FIG. 3.

Referring to FIG. 9, according to an embodiment, in operation 920, the electronic device may output torque having first strength and based on a second direction different from a first direction of the rotational motion of the j oint, by using the actuator. For example, according to an embodiment, the electronic device may adjust strength of the rotational motion of the joint to the first strength indicated by information associated with the user, by using the actuator corresponding to the user's j oint. The first strength of the torque outputted by the electronic device using the actuator may be determined based on information (e.g., the target weight) inputted from the user. For example, the electronic device may obtain information associated with the first strength from the user, by using a visual object being displayed on an external electronic device, such as the text box 426 of FIG. 4. For example, the first strength of the torque outputted by the electronic device may correspond to the strength X of the graphs 530, 630, 730, and 810 of FIGS. 5 to 8.

According to an embodiment, outputting the torque based on operation 920 by the electronic device may be performed based on the mode 320 of FIG. 3. The electronic device may output torque based on operation 920, at a part of the entire time section for performing the strength exercise. For example, while the user performs rotational motion based on a first direction for changing a posture of the joint to a preset posture such as a standby posture, as shown in a time section between the moment t1 and the moment t2 of FIG. 5, the electronic device may output torque based on operation 920. Hereinafter, outputting the torque by the electronic device may be performed based on the actuator, while the user performs the rotational motion based on the first direction. As the electronic device outputs torque based on a second direction (e.g., a second direction corresponding to a direction opposite to the first direction) different from the first direction to the joint by using an actuator, the user's quantity of motion may be increased. For example, torque of operation 920 may correspond to torque resisting the user's rotational motion.

Referring to FIG. 9, according to an embodiment, in operation 930, the electronic device may identify a parameter indicating rotational motion changed by the torque using at least one sensor. For example, according to an embodiment, after adjusting strength of the rotational motion based on the first strength, the electronic device may identify data (e.g., the parameter) indicating rotational motion of the joint, by using the IMU sensor. For example, performing operation 930 by the electronic device is not limited to the order of the flowchart of FIG. 9, and after being activated, the electronic device may continuously monitor the parameter based on operation 930. For example, the electronic device may maintain identifying the parameter based on operation 930 while simultaneously performing other operations of FIG. 9. The parameter identified by the electronic device may include, for example, a first parameter indicating an angle of the joint performing the rotational motion and a second parameter indicating an angular speed of the joint by the rotational motion. When the electronic device has a structure for outputting torque to the user's hip joint based on an embodiment of FIG. 2, the electronic device may identify parameters indicating an angle of the hip joint (e.g., an angle between two body parts such as the waist and thigh centering on the hip joint) and an angular speed of the hip joint.

According to an embodiment, when the electronic device is connected to, for example, the external electronic device 194 of FIG. 2, the electronic device may additionally obtain a parameter associated with the user's heart rate based on operation 930. In an embodiment, the electronic device may further include a sensor for measuring the heart rate. In this case, the parameter identified by the electronic device based on operation 930 may further include a parameter indicating the user's heart rate independently of the external electronic device.

Referring to FIG. 9, according to an embodiment, in operation 940, the electronic device may determine whether a first preset condition for changing strength of torque is satisfied based at least on the parameter of operation 930. The first preset condition may be determined by the electronic device before identifying the rotational motion of operation 910, based on information inputted from the user. For example, the first preset condition may include a condition for adjusting the strength of the torque outputted based on operation 920 to other strength different from the first strength of operation 920, as shown in Table 1. For example, according to an embodiment, the electronic device may identify whether the rotational motion of the joint has been ceased during a preset duration by using data identified based on operation 930. For example, when at least one of the conditions A1 and A2 associated with the angle of Table 1 and at least one of the conditions B1 and B2 associated with the angular speed are satisfied, the electronic device may determine that the first preset condition is satisfied. For example, in response to identifying that at least one of the conditions C1 and C2 associated with the heart rate of Table 1 and at least one of the conditions A1, A2, B1, and B2 associated with the angle and the angular speed are satisfied, the electronic device may determine that the first preset condition is satisfied. Before satisfying the first preset condition (operation 940-NO), the electronic device may maintain identifying a parameter based on operation 930. For example, while outputting the torque of operation 920, the electronic device may maintain identification of the parameter. Failure to satisfy the first preset condition may mean that the user wearing the electronic device normally performs the strength exercise based on quantity of motion increased due to the torque of operation 920.

In operation 950, according to an embodiment, in response to identifying that the first preset condition of operation 940 is satisfied (operation 940-YES), the electronic device may change the strength of the torque to second strength less than the first strength. For example, based at least on identifying that the rotational motion of the joint is ceased during a preset duration based on the identified data of operation 930, the electronic device may adjust the strength of the rotational motion from the first strength to the second strength different from the first strength. For example, the electronic device may perform operation 950 according to switching from the mode 320 of FIG 3 to the mode 330. For example, operation 950 may be performed by the electronic device switched to the mode 330. Changing the strength of the torque based on operation 950 by the electronic device may be performed while maintaining a direction (e.g., a direction based on the second direction) of the torque of operation 920. For example, the torque outputted by the electronic device based on operation 950 may have the second strength and the direction based on the second direction of operation 920. The second strength may be strength obtained by applying a preset ratio to the first strength, and/or may correspond to the strength Y of the graphs 630 and 730 of FIGS. 6 to 7. As the electronic device performs operation 950, the strength of the torque may be reduced while the direction of the torque outputted to the joint is maintained. For example, the torque of operation 950 may correspond to torque resisting the user's rotational motion with the second strength less than the first strength of the torque of operation 920.

Referring to FIG. 9, according to an embodiment, in operation 960, the electronic device may determine whether the second preset condition for changing the direction of the torque is satisfied. For example, the electronic device may maintain identifying one or more parameters indicating the rotational motion based on operation 930, independent of changing the strength of the torque based on operation 950. The electronic device may determine whether the second preset condition is satisfied based on one or more parameters, which are identified after changing the strength of the torque and indicate the rotational motion. For example, after changing the torque strength to the second strength based on operation 950, the electronic device may identify whether the rotational motion of the joint is ceased again during a preset duration.

In an embodiment, a second preset condition of operation 960 may be determined by the electronic device before identifying the rotational motion of operation 910, based on information inputted from the user. For example, the second preset condition may include a condition for changing a direction of the torque being outputted based on at least one of the operations 920 and 950 to a direction, which is based on the first direction different from the second direction of the operations 920 and 950, as shown in Table 2. For example, in response to identifying that at least one of the condition A3 associated with the angle and the condition B3 associated with the angular speed in Table 2 is satisfied, the electronic device may determine that the second preset condition is satisfied. Before the second preset condition is satisfied (operation 960-NO), the electronic device may maintain outputting the torque having the second strength, based on operation 950. Failure to satisfy the second preset condition may mean that the user wearing the electronic device normally performs the strength exercise based on the quantity of motion increased due to the torque having the changed second strength of the operation 950.

In operation 970, according to an embodiment, in response to identifying that the second preset condition of operation 960 is satisfied (960-YES), the electronic device may change the direction of the torque to a direction based on the first direction. For example, the electronic device may perform operation 970 according to switching from the mode 330 of FIG. 3 to the mode 340. For example, operation 970 may be performed by the electronic device switched to the mode 340. Changing the direction of the torque based on operation 970 by the electronic device may include changing the direction of the torque in a direction based on the first direction of the rotational motion performed by the user to change a posture of the joint to a preset posture such as a standby posture. As the electronic device outputs the torque based on the first direction, the user's quantity of motion may be reduced. For example, the user may more easily switch a user's posture to a preset posture such as the standby posture, based on the torque of operation 970.

According to an embodiment, in a state of changing a direction of the torque based on operation 970, the electronic device may change the strength of the torque to third strength independent of the first strength and the second strength of operations 920 and 950. The third strength may correspond to predetermined strength so that the user may more easily perform restoring to the standby posture. For example, the third strength may be determined based on data such as the user's weight or height. For example, the third strength may correspond to the strength Z of the graph 730 of FIG. 7.

As described above, according to an embodiment, the electronic device may output torque having strength and/or a direction adjusted based on operations 920, 950, and 980 to the user performing the strength exercise. Adjusting the strength and/or direction of the torque by the electronic device may be performed dynamically based on preset conditions of operations 940 and 960. The preset conditions of operations 940 and 960 may correspond to a condition for determining a failure moment of the user performing the strength exercise. For example, when the user reaches a threshold point before performing the strength exercise as many times as the number inputted to the electronic device, the electronic device may identify that the user has reached the threshold point based on operations 940 and 960. In response to identifying that the user has reached the threshold point, the electronic device may adjust the strength and/or direction of the torque to guide the user to complete the strength exercise based on the number of times.

Referring to FIG. 9, according to an embodiment, in operation 980, the electronic device may determine whether a preset user input for ceasing the rotational motion is received. For example, the user input may include an operation of pressing a preset button (e.g., a stop button) included in the electronic device, and/or an operation of performing a preset comment (e.g., a preset comment such as "I will stop exercising"). For example, the electronic device may output a UI for receiving a user input of operation 980, based on the number of repetitions received together with the target weight from the user. For example, the UI may include an operation of displaying a preset screen (e.g., a screen including preset text such as "Do you want to stop exercising?") through a display of an external electronic device (e.g., the external electronic devices 192, 194, and 196 of FIG. 1) different from the electronic device, and/or an operation of displaying an acoustic signal (e.g., a preset comment such as "The target number has been reached. Do you want to rest?") outputted through a speaker (e.g., the speaker 160 of FIG. 1) of the electronic device.

Before receiving the user input of operation 980 (operation 980-NO), the electronic device may maintain outputting the torque based on operation 970. For example, independent of performing the user input of operation 980, when the user continuously performs the strength exercise, the electronic device may maintain outputting the torque that assists changing to the standby posture based on operation 970.

In operation 990, according to an embodiment, in response to receiving the preset user input of operation 980 (operation 980-YES), the electronic device may cease outputting the torque using the actuator. For example, the electronic device may cease outputting the torque corresponding to the user's rotational motion for restoring to a preset posture such as the standby posture, such as operations 920, 950, and 970. For example, the electronic device may cease outputting the torque using the actuator, by minimizing the amount of power supplied to the actuator.

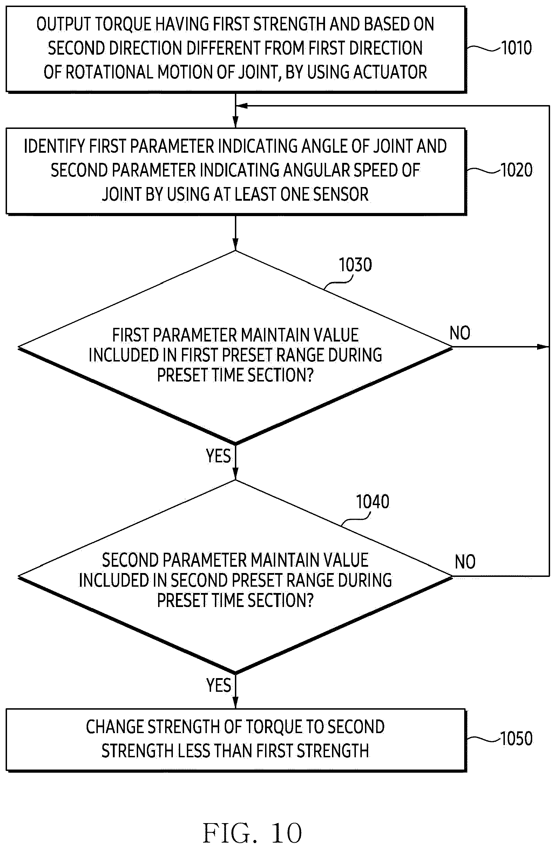

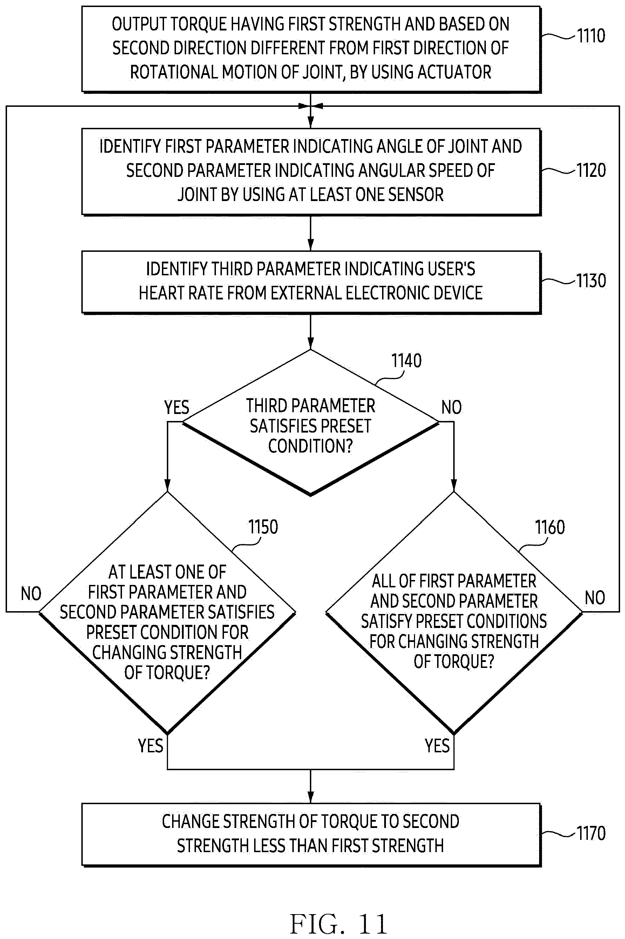

Hereinafter, an example of operations 920, 930, 940, and 950 of FIG. 9 will be described in detail with reference to FIG. 10 and/or FIG. 11.

FIG. 10 is a flowchart illustrating an operation performed by an electronic device to increase a user's quantity of motion by rotational motion, according to an embodiment. The electronic device of FIG. 10 may correspond to an example of the electronic device 101 of FIGS. 1 to 2, 5 to 8, and/or the electronic device of FIGS. 3 to 4 and/or 9. For example, at least one of operations of FIG. 10 may be performed by the electronic device 101 of FIG. 1 and/or the processor 110 of FIG. 1. For example, operations 920, 930, 940, and 950 of FIG. 9 may be related to at least one of the operations of FIG. 10. Hereinafter, descriptions overlapped those of FIG. 9 are omitted.

Referring to FIG. 10, according to an embodiment, in operation 1010, the electronic device may output torque having first strength and based on a second direction different from a first direction of rotational motion of a joint, by using an actuator. For example, the electronic device may perform operation 1010 similarly to operation 920 of FIG. 9.

Referring to FIG. 10, according to an embodiment, in operation 1020, the electronic device may identify a first parameter indicating an angle of a joint and a second parameter indicating an angular speed of the joint by using at least one sensor. Performing operation 1020 by the electronic device may be performed similarly to operation 930 of FIG. 9. For example, each of the first parameter and the second parameter identified by the electronic device may be identified through at least one sensor while the user performs strength exercise, as shown in the graphs 610 and 620 of FIG. 6.

Referring to FIG. 10, according to an embodiment, in operation 1030, the electronic device may determine whether the first parameter maintains a value included in a first preset range during a preset time section. For example, the electronic device may identify whether the angle of the joint identified based on operation 1020 is included within the first preset range during the preset time section. The preset time section and the first preset range of operation 1030 may be associated with a preset condition for determining a failure moment of the user performing the strength exercise. For example, the first preset range may include an angle different from the angle of the user's joint (e.g., 180°) in the standby posture. For example, the preset time section and the preset range of the conditions A1 and A2 in Table 1 may correspond to an example of each of the preset time section and the first preset range of operation 1030.

In operation 1040, according to an embodiment, in response to identifying that the first parameter maintains a value included in the first preset range during a preset time section (operation 1030-YES), the electronic device may determine whether the second parameter maintains a value included in the second preset range during the preset time section. For example, the electronic device may identify whether the angular speed of the joint identified based on operation 1020 is included within the second preset range during the preset time section of operation 1030. The preset time section and the second preset range of operation 1040 may be associated with a preset condition for determining a failure moment of the user performing the strength exercise. For example, the second preset range may include an angular speed indicating that the user's rotational motion is substantially ceased. For example, the preset time section of the conditions B1 and B2 in Table 1 may correspond to an example of the preset time section of operation 1040. For example, a range distinguished by the preset range and/or the preset threshold of the conditions B1 and B2 in Table 1 may correspond to an example of the second preset range of operation 1040.

Referring to FIG. 10, when the first parameter is not included in the first preset range during the preset time section (operation 1030-NO), or the second parameter is not included in the second preset range during the preset time section (operation 1040-NO), the electronic device may maintain identifying the first parameter and the second parameter based on operation 1020. According to an embodiment, the electronic device may maintain outputting the torque of the operation 1010.

Referring to FIG. 10, when the first parameter is included in the first preset range during the preset time section (operation 1030-YES), and the second parameter is included in the second preset range during the preset time section (1040-YES), in operation 1050, according to an embodiment, the electronic device may change strength of the torque to the second strength less than the first strength. Operation 1050 may be performed by the electronic device in response to identifying that it is impossible for a user with a posture different from the standby posture to perform rotational motion based on the strength exercise. The user having a posture different from the standby posture may be identified by the electronic device performing operation 1030. Impossible of the user performing the rotational motion may be resolved by the electronic device according to performing operation 1040. According to an embodiment, the electronic device may perform operation 1050 similarly to operation 950 of FIG. 9.

As described above, according to an embodiment, in response to identifying a change in the angle and angular speed indicating a failure moment, the electronic device may reduce the strength of the torque outputted to the user's joint. As the strength of the torque is reduced, the user may complete the strength exercise based on the reduced quantity of motion. Hereinafter, an operation performed by the electronic device in an embodiment in which the electronic device identifies a parameter in a type different from the first and second parameters of FIG. 10 from the external electronic device will be described in detail with reference to FIG. 11.

FIG. 11 is a flowchart illustrating an operation performed by an electronic device to maintain a user's rotational motion, according to an embodiment. The electronic device of FIG. 11 may correspond to an example of the electronic device 101 of FIGS. 1 to 2, 5 to 8, and/or the electronic device of FIGS. 3 to 4 and/or 9 to 10. For example, at least one of operations of FIG. 11 may be performed by the electronic device 101 of FIG. 1 and/or the processor 110 of FIG. 1. For example, operations 920, 930, 940, and 950 of FIG. 9 may be related to at least one of the operations of FIG. 11. Hereinafter, descriptions overlapped those of FIGS. 9 to 10 are omitted.

Referring to FIG. 11, according to an embodiment, in operation 1110, the electronic device may output torque having first strength and based on second direction different from first direction of rotational motion of a joint, by using an actuator. For example, the electronic device may perform operation 1110 similarly to operation 920 of FIG. 9 and/or operation 1010 of FIG. 10.

Referring to FIG. 11, according to an embodiment, in operation 1120, the electronic device may identify a first parameter indicating an angle of a joint and a second parameter indicating an angular speed of the joint by using at least one sensor. For example, the electronic device may perform operation 1120 similarly to operation 930 of FIG. 9 and/or operation 1020 of FIG. 10.

Referring to FIG. 11, according to an embodiment, in operation 1130, the electronic device may identify a third parameter indicating a user's heart rate from an external electronic device. For example, the electronic device may be connected with an external electronic device including a sensor capable of measuring a user's heart rate, such as the external electronic device 192 and/or the external electronic device 194 of FIG. 1. In response to identifying the external electronic device including the sensor capable of measuring the user's heart rate, the electronic device may perform at least one of operations of FIG. 11, including operation 1130.

Referring to FIG. 11, according to an embodiment, in operation 1140, the electronic device may determine whether the third parameter of operation 1130 satisfies a preset condition. For example, the electronic device may determine whether the user's heart rate identified based on operation 1120 increases based on an increase rate greater than or equal to a preset increase rate, or exceeds a preset threshold. The preset condition of operation 1140 may be associated with a preset condition for determining a failure moment of a user performing strength exercise. For example, the conditions C1 and C2 of Table 1 may correspond to an example of the preset condition of operation 1140.

In response to identifying that the third parameter satisfies the preset condition (operation 1140-YES), in operation 1150, according to an embodiment, the electronic device may determine whether at least one of the first parameter and the second parameter satisfies a preset condition for changing strength of torque. For example, the electronic device may determine whether an angle of a joint indicated by the first parameter satisfies a preset condition (e.g., operation 1030 of FIG. 10 and/or conditions A1 and A2 of Table 1). For example, the electronic device may determine whether an angular speed of the joint indicated by the second parameter satisfies a preset condition (e.g., operation 1040 of FIG. 10 and/or conditions B1 and B2 of Table 1). When neither the first parameter nor the second parameter satisfies the preset condition according to operation 1150 (operation 1150-NO), the electronic device may maintain identifying the first to third parameters based on operations 1120 and 1130. Maintaining identifying the first to third parameters based on operations 1120 and 1130 by the electronic device may be performed while outputting the torque of operation 1110.

In response to identifying that the third parameter does not satisfy the preset condition (operation 1140-NO), in operation 1160, according to an embodiment, the electronic device may determine whether all of the first parameter and the second parameter satisfy preset conditions for changing the strength of the torque. According to an embodiment, the electronic device may perform operation 1160 similarly to operations 1030 and 1040 of FIG. 10. When neither the first parameter nor the second parameter satisfies the preset condition (operation 1160-NO) according to operation 1160, the electronic device may identify the first to third parameters based on operations 1120 and 1130.

When at least one parameter satisfying the preset condition is identified from among the first parameter or the second parameter according to operation 1150 (operation 1150-YES), or all of the first parameter and the second parameter satisfy the preset conditions according to operation 1160 (operation 1160-YES), in operation 1170, according to an embodiment, the electronic device may change the strength of the torque to the second strength less than the first strength. For example, the electronic device may perform operation 1170 similarly to operation 950 of FIG. 9 and/or operation 1050 of FIG. 10.

As described above, according to an embodiment, in response to identifying a change in the heart rate indicating a failure moment, the electronic device may ease determining the failure moment based on the angle and angular speed. For example, unlike both the angle and angular speed satisfy the preset condition associated with the failure moment, as shown in FIG. 10, the electronic device may reduce the strength of the torque outputted to the joint, based on whether the angle or angular speed satisfies the preset condition associated with the failure moment, in response to identifying a change in the heart rate indicating the failure moment.

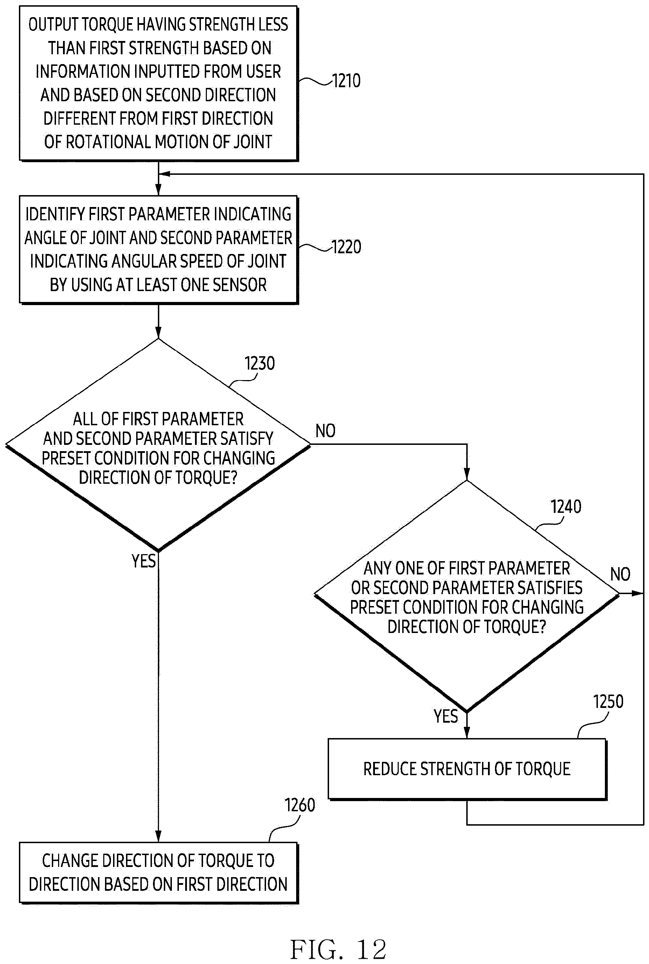

As described above, according to an embodiment, in response to identifying a change in the angle and angular speed indicating the failure moment, the electronic device may reduce the strength of the torque outputted to the user's j oint. As the strength of the torque decreases, the user may complete the strength exercise based on the reduced quantity of motion. Hereinafter, an operation performed by the electronic device in response to identifying coming of additional failure moments after reducing the strength of the torque will be described in detail with reference to FIG. 12.

FIG. 12 is a flowchart illustrating an operation performed by an electronic device to guide a user's rotational motion, according to an embodiment. The electronic device of FIG. 12 may correspond to an example of the electronic device 101 of FIGS. 1 to 2, 5 to 8, and/or the electronic device of FIGS. 3 to 4 and/or 9 to 11. For example, at least one of operations of FIG. 12 may be performed by the electronic device 101 of FIG. 1 and/or the processor 110 of FIG. 1. For example, operations 950, 960, and 970 of FIG. 9 may be related to at least one of the operations of FIG. 12. Hereinafter, descriptions overlapped those of FIGS. 9 to 11 are omitted.

Referring to FIG. 12, according to an embodiment, in operation 1210, the electronic device may output torque having strength less than first strength based on information inputted from a user and based on a second direction different from a first direction of rotational motion of ajoint. For example, the electronic device may perform operation 1210 similarly to operation 950 of FIG. 9, operation 1050 of FIG. 10, and/or operation 1170 of FIG. 11. For example, operation 1210 may be performed by the electronic device switched to the mode 330 of FIG. 3.

Referring to FIG. 12, in operation 1220, according to an embodiment, the electronic device may identify a first parameter indicating an angle of a joint and a second parameter indicating an angular speed of the joint by using at least one sensor. For example, the electronic device may perform operation 1220 similarly to operation 1020 of FIG. 10 and/or operation 1120 of FIG. 11.

Referring to FIG. 12, according to an embodiment, in operation 1230, the electronic device may determine whether all of the first parameter and the second parameter satisfy a preset condition for changing a direction of the torque. The preset condition may be associated with a preset condition for determining a failure moment of a user performing strength exercise. For example, the electronic device may identify that an angle indicated by the first parameter is maintained during a preset time section within a preset range based on an angle different from a preset angle corresponding to a standby posture. For example, the electronic device may identify that an angular speed indicated by the second parameter is maintained during a preset time section within a preset range indicating that rotational motion associated with the strength exercise is ceased. For example, the condition A3 associated with the angle and the condition B3 associated with the angular speed in Table 2 may correspond to an example of the preset condition of operation 1230.

When the first and second parameters do not simultaneously satisfy the preset condition for changing the direction of the torque (operation 1230-NO), in operation 1240, according to an embodiment, the electronic device may determine whether any one of the first parameter or the second parameter satisfies the preset condition for changing the direction of the torque. A preset condition of operation 1240 may correspond to the preset condition of operation 1230. According to an embodiment, the electronic device may identify the number of parameters satisfying the preset condition from among the first parameter and the second parameter, based on operations 1230 and 1240. For example, operation 1230 may correspond to an operation of determining whether the number of parameters satisfying the preset condition from among the first parameter and the second parameter is 2 or more. For example, operation 1240 may correspond to an operation of determining whether the number of parameters satisfying the preset condition from among the first parameter and the second parameter corresponds to 1.

When neither the first parameter nor the second parameter satisfies the preset condition for changing the direction of the torque (operation 1240-NO), according to an embodiment, the electronic device may maintain identifying the first parameter and the second parameter based on operation 1220. Maintaining identifying the first parameter and the second parameter by the electronic device may be performed while maintaining outputting of the torque based on operation 1210.

In response to identifying that one of the first parameter and the second parameter satisfies the preset condition of operation 1240 (operation 1240-YES), in operation 1250, according to an embodiment, the electronic device may reduce the strength of the torque. For example, the electronic device may reduce the strength of the torque below the strength of operation 1210. As the electronic device repeatedly performs operation 1250 based on operations 1240, the strength of the torque outputted by the electronic device to the joint by using the actuator may be gradually reduced. Referring to FIG. 12, the electronic device performing operation 1250 may continuously perform operations 1220, 1230, and 1240. For example, the electronic device may output the torque adjusted based on operation 1250 and maintain identifying the first to second parameters based on operation 1220 at the same time.

When the first parameter and the second parameter simultaneously satisfy the preset condition for changing the direction of the torque (operation 1230-YES), in operation 1260, according to an embodiment, the electronic device may change the direction of the torque to a direction based on the first direction. For example, independent of outputting the torque based on the second direction of operation 1210, the electronic device may switch the direction of the torque to the direction based on the first direction based on operation 1260. As described above in operation 910, the first direction may correspond to a direction of the user's rotational motion for restoring to the standby posture. As the electronic device switches the direction of the torque outputted using the actuator from the direction based on the second direction of operation 1210 to the direction based on the first direction, the electronic device may output torque promoting the user's rotational motion of for restoring to the standby posture. For example, the electronic device may perform operation 1260 according to switching to the mode 340 of FIG. 3. For example, the electronic device may perform operation 1260 similarly to operation 970 of FIG. 9. For example, according to an embodiment, after operation 1260, the electronic device may perform operations (e.g., operations 980 and 990) after operation 970 of FIG. 9.

Hereinafter, an operation performed by the electronic device according to an embodiment in response to identifying that a user falls will be described in detail with reference to FIG. 13.

FIG. 13 is a flowchart illustrating an operation in which an electronic device ceases outputting a torque using an actuator, according to an embodiment. The electronic device of FIG. 13 may correspond to an example of the electronic device 101 of FIGS. 1 to 2, 5 to 8, and/or the electronic device of FIGS. 3 to 4 and/or 9 to 12. For example, at least one of operations of FIG. 13 may be performed by the electronic device 101 of FIG. 1 and/or the processor 110 of FIG. 1. For example, at least one of the operations of FIG. 13 may be performed independently of the operations of FIGS. 9 to 12.

Referring to FIG. 13, according to an embodiment, in operation 1310, the electronic device may output torque by using an actuator. For example, the operation 1310 may include operations 920, 950, and 970 of FIG. 9, operations 1010 and 1050 of FIG. 10, operations 1110 and 1170 of FIG. 11, and/or operations 1210, 1250, and 1260 of FIG. 12. For example, in modes 320, 330, and 340 different from the mode 310 corresponding to the manual mode of FIG. 3, the electronic device may perform operations of FIG. 13, including an operation 1310.

Referring to FIG. 13, according to an embodiment, in operation 1320, the electronic device may identify a parameter indicating motion of the electronic device. For example, the electronic device may identify one or more parameters indicating the motion of the electronic device using at least one sensor (e.g., the sensor 150 of FIG. 1). The electronic device may identify one or more parameters indicating a direction and/or magnitude of acceleration applying on the electronic device, using the IMU sensor.

Referring to FIG. 13, according to an embodiment, in operation 1330, the electronic device may determine whether other motion different from rotational motion of a j oint is identified based on the parameter identified based on the operation 1320. For example, the other motion is motion different from the user's motion (e.g., the rotational motion) associated with the strength exercise, and may correspond to the motion in which the user falls like the motion of the user after the moment t2 of FIG. 8. Before identifying the other motion (operation 1330-NO), the electronic device may maintain identifying other motion based on the operations 1320 and 1330.

According to an embodiment, in response to identifying other motion different from the rotational motion of the joint (operation 1330-YES), in operation 1340, the electronic device may at least temporarily cease outputting the torque based on operation 1310. For example, the electronic device may cease outputting torque interfering or promoting the rotational motion of the joint corresponding to the actuator based on operation 1310. As the electronic device ceases outputting the torque based on the operation 1340, the user's injury due to a combination of the torque of operation 1310 and the other motion (e.g., user's fall) may be prevented. For example, operation 1340 may be performed by the electronic device switched to the mode 310 of FIG. 3.

Referring to FIG. 13, the electronic device may perform operations 1320, 1330, and 1340 by using a process (e.g., thread) executed in the electronic device independent of the operations of FIGS. 9 to 12. In this case, according to an embodiment, the electronic device may correspond to the user's fall occurring while the user performs the strength exercise.

As described above, according to an embodiment, the electronic device may assist the user's strength exercise based on a target weight received from the user (e.g., the mode 320 in FIG. 3). For example, assisting the strength exercise by the electronic device may be performed by outputting torque of strength corresponding to the target weight to the user's one or more joints, by using one or more actuators corresponding to each of the one or more joints. In response to identifying that the user has reached a failure moment, the electronic device may output the torque based on a weight below the target weight so that the strength exercise is continuously performed (e.g., the mode 330 in FIG. 3). Alternatively, the electronic device may output torque promoting a user's motion based on the strength exercise (e.g., the mode 340 of FIG. 3). Adjusting the torque associated with the strength exercise by the electronic device may be performed based on at least one of an angle, angular speed of one or more joints, which is identified by using at least one sensor, or user-related data (e.g., data indicating heart rate) received from an external electronic device connected to the electronic device.

As described above, according to an embodiment, an electronic device may comprise an actuator associated with a joint of a user of the electronic device, at least one sensor, memory storing one or more instructions, and a processor operatively coupled with the actuator, the at least one sensor, and the memory. The processor may be configured to, when executing the one or more instructions, adjust strength of rotational motion of the joint as first strength indicated by information associated with the user by using the actuator. The processor may be configured to, when executing the one or more instructions, after adjusting the strength of the rotational motion to the first strength, identify data indicating the rotational motion of the joint by using the at least one sensor. The processor may be configured to, when executing the one or more instructions, at least based on identifying the rotational motion is ceased for a preset duration based on the identified data, adjust the strength of the rotational motion from the first strength to second strength different from the first strength.

For example, the processor may be configured to, when executing the one or more instructions, identify the data indicating at least one of an angle or angular speed of the joint by using the at least one sensor corresponding to an inertial measurement unit (IMU) sensor to estimate motion of the electronic device caused by the rotational motion of the j oint. The processor may be configured to, when executing the one or more instructions, in response to identifying that the rotational motion is ceased for the preset duration based on at least one of the angle or the angular speed indicated by the data, adjust the strength of the rotational motion from the first strength to the second strength.

For example, the electronic device may further comprise a communication circuitry to communicate with an external electronic device. The processor may be configured to, when executing the one or more instructions, identify a heart rate of the user from the external electronic device and adjust the strength of the rotational motion from the first strength to the second strength different from the first strength based on the identified data or the heart rate.

For example, the processor may be configured to, when executing the one or more instructions, at least based on identifying that the rotational motion is ceased for another preset duration different from the preset duration after adjusting the strength of the rotational motion to the second strength, adjust the strength of the rotational motion to third strength that is different from the second strength to maintain a posture of the joint as a first preset posture.

For example, the processor may be configured to, when executing the one or more instructions, in a first time section that the posture of the joint is changed from the first posture to a second posture by the rotational motion, adjust the strength of the rotational motion to the first strength. The processor may be configured to, when executing the one or more instructions, in a second time section different from the first time section that the posture of the joint is changed from the second posture to the first posture by the rotational motion, cease to adjust the strength of the rotational motion at least temporarily.

For example, the processor may be configured to, when executing the one or more instructions, in response to identifying that a number by which the posture of the joint is periodically changed by the rotational motion is increased to a preset number indicated by the information after adjusting the strength of the rotational motion to the first strength, adjust the strength of the rotational motion to the second strength different from the first strength.

For example, the electronic device may further comprise a speaker. The processor may be configured to, when executing the one or more instructions, after changing the strength of the rotational motion to the second strength, output an acoustic signal to guide the rotational motion through the speaker.

For example, the processor may be configured to, when executing the one or more instructions, while adjusting the strength of the rotational motion to the first strength or the second strength, identify other data indicating motion of the electronic device by using the at least one sensor. The processor may be configured to, when executing the one or more instructions, in response to identifying occurrence of other motion different from the rotational motion based on the other data, cease to adjust the strength of the rotational motion to the first strength or the second strength at least temporarily.

For example, the processor may be configured to, when executing the one or more instructions, in response to identifying that the identified parameter is maintained within the preset range after adjusting the strength of the rotational motion to the second strength smaller than the first strength, adjust third strength smaller than the second strength.

As described above, according to an embodiment, a method of an electronic device may comprise, by using at least one sensor included in the electronic device, identifying rotational motion rotated along a first direction at a joint of a user of the electronic device. The method may comprise, in response to identifying the rotational motion of the joint, outputting torque having first strength and based on a second direction different from the first direction, by using an actuator included in the electronic device and corresponding to the j oint. The method may comprise, while outputting the torque by using the actuator, identifying data indicating the rotational motion changed by the torque by using the at least one sensor. The method may comprise, at least based on identifying the data indicating the rotational motion maintained within a first range during a first time section, changing strength of the torque to second strength lower than the first strength by using the actuator. The method may comprise, at least based on identifying the data indicating the rotational motion that is maintained within a second range during a second time section while outputting the torque based on the second direction and having the second strength, changing a direction of the torque to a direction based on the first direction among the first direction and the second direction, by using the actuator.

For example, the method may comprise, in response to identifying other rotational motion of the joint rotated along the second direction different from the first direction, ceasing to output the torque by using the actuator.

For example, the outputting the torque may comprise outputting the torque based on the first strength indicated by information associated with the user, in memory of the electronic device. The changing the strength of the torque to the second strength may comprise, in response to identifying that a number by which a posture of the joint is changed to a preset posture along the first direction is increased to a preset number indicated by the information while outputting the torque having the first strength, changing the first strength to the second strength.

For example, the identifying the parameter may comprise identifying a first parameter indicating an angle of the joint and a second parameter indicating an angular speed of the joint. The changing the torque to the second strength may comprise, in response to identifying that the first parameter is included within the first range during the first time section and that the second parameter is included within a third range during the first time section, adjusting the strength of the torque to the second strength.

For example, the identifying the parameter may comprise identifying a third parameter indicating a heart rate of the user from an external electronic device different from the electronic device by using a communication circuitry included in the electronic device. The changing the strength of the torque to the second strength may comprise, in response to identifying that the third parameter is maintained within a fourth range during the first time section and that the first parameter is maintained within the first range during the first time section, changing the strength of the torque to the second torque. The changing the strength of the torque to the second strength may comprise, in response to identifying that the third parameter is maintained within the fourth range during the first time section and that the second parameter is maintained within the third range during the first time section, changing the strength of the torque to the second strength.

For example, the method of the electronic device may comprise, while outputting the torque having the second strength and based on the second direction, initiating output of a preset acoustic signal through a speaker included in the electronic device.

As described above, according to an embodiment, a method of an electronic device may comprise adjusting strength of rotational motion of the joint as first strength indicated by information associated with the user by using an actuator associated with a joint of a user of the electronic device. The method may comprise, after adjusting the strength of the rotational motion to the first strength, identifying data indicating the rotational motion of the joint by using the at least one sensor included in the electronic device. The method may comprise at least based on identifying the rotational motion is ceased for a preset duration based on the identified data, adjusting the strength of the rotational motion from the first strength to second strength different from the first strength.

For example, identifying the parameter may comprise identifying the data indicating at least one of an angle or angular speed of the joint by using an inertial measurement unit (IMU) sensor to estimate motion of the electronic device caused by the rotational motion of the joint. Identifying the parameter may comprise, in response to identifying that the rotational motion is ceased for the preset duration based on at least one of the angle or the angular speed indicated by the data, adjusting the strength of the rotational motion from the first strength to the second strength.

For example, the method of the electronic device may comprise, at least based on identifying that the rotational motion is ceased for another preset duration different from the preset duration after adjusting the strength of the rotational motion to the second strength, adjusting the strength of the rotational motion to third strength that is different from the second strength to maintain a posture of the joint as a first preset posture.

For example, the method of the electronic device may comprise, in a first time section that the posture of the joint is changed from the first posture to a second posture by the rotational motion, adjusting the strength of the rotational motion to the first strength. The method may comprise, in a second time section different from the first time section that the posture of the joint is changed from the second posture to the first posture by the rotational motion, ceasing to adjust the strength of the rotational motion at least temporarily.

For example, the method of the electronic device may comprise, in response to identifying that the identified parameter is maintained within the preset range after adjusting the strength of the rotational motion to the second strength smaller than the first strength, adjusting third strength smaller than the second strength.

As described above, according to an embodiment, an electronic device may comprise an actuator associated with a joint of a user of the electronic device, at least one sensor, memory storing one or more instructions, and a processor operatively coupled with the actuator, the at least one sensor, and the memory. The processor may be configured to, when executing the one or more instructions, by using the actuator least one sensor included in the electronic device, identify rotational motion rotated along a first direction at a joint of a user of the electronic device. The processor may be configured to, when executing the one or more instructions, in response to identifying the rotational motion of the joint, output torque having first strength and based on a second direction different from the first direction, by using the actuator. The processor may be configured to, when executing the one or more instructions, while outputting the torque by using the actuator, identify data indicating the rotational motion changed by the torque by using the at least one sensor. The processor may be configured to, when executing the one or more instructions, at least based on identifying the data indicating the rotational motion maintained within a first range during a first time section, change strength of the torque to second strength lower than the first strength by using the actuator. The processor may be configured to, when executing the one or more instructions, at least based on identifying the data indicating the rotational motion that is maintained within a second range during a second time section while outputting the torque based on the second direction and having the second strength, change a direction of the torque to a direction based on the first direction among the first direction and the second direction, by using the actuator.

The apparatus described above may be implemented as a combination of hardware components, software components, and/or hardware components and software components. For example, the devices and components described in the embodiments may be implemented using one or more general purpose computers or special purpose computers such as processors, controllers, arithmetical logic unit(ALU), digital signal processor, microcomputers, field programmable gate array (FPGA), PLU(programmable logic unit), microprocessor, any other device capable of executing and responding to instructions. The processing device may perform an operating system OS and one or more software applications performed on the operating system. In addition, the processing device may access, store, manipulate, process, and generate data in response to execution of the software. For convenience of understanding, although one processing device may be described as being used, a person skilled in the art may see that the processing device may include a plurality of processing elements and/or a plurality of types of processing elements. For example, the processing device may include a plurality of processors or one processor and one controller. In addition, other processing configurations, such as a parallel processor, are also possible.

The software may include a computer program, code, instruction, or a combination of one or more of them and configure the processing device to operate as desired or command the processing device independently or in combination. Software and/or data may be embodied in any type of machine, component, physical device, computer storage medium, or device to be interpreted by a processing device or to provide instructions or data to the processing device. The software may be distributed on a networked computer system and stored or executed in a distributed manner. Software and data may be stored in one or more computer-readable recording media.

The method according to the embodiment may be implemented in the form of program instructions that may be performed through various computer means and recorded in a computer-readable medium. In this case, the medium may continuously store a computer-executable program or temporarily store the program for execution or download. In addition, the medium may be a variety of recording means or storage means in which a single or several hardware are combined and is not limited to media directly connected to any computer system and may be distributed on the network. Examples of media may include magnetic media such as hard disks, floppy disks and magnetic tapes, optical recording media such as CD-ROMs and DVDs, magnetooptical media such as floptical disks, ROMs, RAMs, flash memories, and the like to store program instructions. Examples of other media include app stores that distribute applications, sites that supply or distribute various software, and recording media or storage media managed by servers.

Although embodiments have been described according to limited embodiments and drawings as above, various modifications and modifications are possible from the above description to those of ordinary skill in the art. For example, even if the described techniques are performed in a different order from the described method, and/or components such as the described system, structure, device, circuit, etc. are combined or combined in a different form from the described method or are substituted or substituted by other components or equivalents, appropriate results may be achieved.

Therefore, other implementations, other embodiments, and equivalents to the claims fall within the scope of the claims to be described later.

## Claims

1. An electronic device comprising:
an actuator associated with a joint of a user of the electronic device;
at least one sensor;
memory storing one or more instructions; and
a processor operatively coupled with the actuator, the at least one sensor, and the memory,
wherein the processor configured to, when executing the one or more instructions:
adjust strength of rotational motion of the joint as first strength indicated by information associated with the user by using the actuator;
after adjusting the strength of the rotational motion to the first strength, identify data indicating the rotational motion of the joint by using the at least one sensor; and
at least based on identifying the rotational motion is ceased for a preset duration based on the identified data, adjust the strength of the rotational motion from the first strength to second strength different from the first strength.

2. The electronic device of claim 1, wherein the processor configured to, when executing the one or more instructions:
identify the data indicating at least one of an angle or angular speed of the joint by using the at least one sensor corresponding to an inertial measurement unit (IMU) sensor to estimate motion of the electronic device caused by the rotational motion of the joint;
in response to identifying that the rotational motion is ceased for the preset duration based on at least one of the angle or the angular speed indicated by the data, adjust the strength of the rotational motion from the first strength to the second strength.

3. The electronic device of any one of preceding claims, further comprising a communication circuitry to communicate with an external electronic device;
wherein the processor configured to, when executing the one or more instructions:
identify a heart rate of the user from the external electronic device;
adjust the strength of the rotational motion from the first strength to the second strength different from the first strength based on the identified data or the heart rate.

4. The electronic device of any one of preceding claims, wherein the processor configured to, when executing the one or more instructions:
at least based on identifying that the rotational motion is ceased for another preset duration different from the preset duration after adjusting the strength of the rotational motion to the second strength, adjust the strength of the rotational motion to third strength that is different from the second strength to maintain a posture of the joint as a first preset posture.

5. The electronic device of any one of preceding claims, wherein the processor configured to, when executing the one or more instructions:
in a first time section that the posture of the joint is changed from the first posture to a second posture by the rotational motion, adjust the strength of the rotational motion to the first strength;
in a second time section different from the first time section that the posture of the joint is changed from the second posture to the first posture by the rotational motion, cease to adjust the strength of the rotational motion at least temporarily.

6. The electronic device of any one of preceding claims, wherein the processor configured to, when executing the one or more instructions:
in response to identifying that a number by which the posture of the joint is periodically changed by the rotational motion is increased to a preset number indicated by the information after adjusting the strength of the rotational motion to the first strength, adjust the strength of the rotational motion to the second strength different from the first strength.

7. The electronic device of any one of preceding claims, further comprising:
a speaker,
wherein the processor configured to, when executing the one or more instructions:
after changing the strength of the rotational motion to the second strength, output an acoustic signal to guide the rotational motion through the speaker.

8. The electronic device of any one of preceding claims, wherein the processor configured to, when executing the one or more instructions:
while adjusting the strength of the rotational motion to the first strength or the second strength, identify other data indicating motion of the electronic device by using the at least one sensor; and
in response to identifying occurrence of other motion different from the rotational motion based on the other data, cease to adjust the strength of the rotational motion to the first strength or the second strength at least temporarily.

9. The electronic device of any one of preceding claims, wherein the processor configured to, when executing the one or more instructions:
in response to identifying that a parameter is maintained within a preset range after adjusting the strength of the rotational motion to the second strength smaller than the first strength, adjust third strength smaller than the second strength.

10. A method of an electronic device comprising:
by using at least one sensor included in the electronic device, identifying rotational motion rotated along a first direction at a joint of a user of the electronic device;
in response to identifying the rotational motion of the joint, outputting torque having first strength and based on a second direction different from the first direction, by using an actuator included in the electronic device and corresponding to the joint;
while outputting the torque by using the actuator, identifying data indicating the rotational motion changed by the torque by using the at least one sensor;
at least based on identifying the data indicating the rotational motion maintained within a first range during a first time section, changing strength of the torque to second strength lower than the first strength by using the actuator; and
at least based on identifying the data indicating the rotational motion that is maintained within a second range during a second time section while outputting the torque based on the second direction and having the second strength, changing a direction of the torque to a direction based on the first direction among the first direction and the second direction, by using the actuator.

11. The method of claim 10, further comprising:
in response to identifying other rotational motion of the joint rotated along the second direction different from the first direction, ceasing to output the torque by using the actuator.

12. The method of any one of preceding claims, wherein the outputting the torque further comprising:
outputting the torque based on the first strength indicated by information associated with the user, in memory of the electronic device,
wherein the changing the strength of the torque to the second strength further comprising:
in response to identifying that a number by which a posture of the joint is changed to a preset posture along the first direction is increased to a preset number indicated by the information while outputting the torque having the first strength, changing the first strength to the second strength.

13. The method of any one of preceding claims, wherein the identifying the parameter further comprising:
identifying a first parameter indicating an angle of the joint and a second parameter indicating an angular speed of the joint,
wherein the changing the torque to the second strength further comprising:
in response to identifying that the first parameter is included within the first range during the first time section and that the second parameter is included within a third range during the first time section, adjusting the strength of the torque to the second strength.

14. The method of any one of preceding claims, wherein the identifying the parameter further comprising:
identifying a third parameter indicating a heart rate of the user from an external electronic device different from the electronic device by using a communication circuitry included in the electronic device;
wherein the changing the strength of the torque to the second strength further comprising:
in response to identifying that the third parameter is maintained within a fourth range during the first time section and that the first parameter is maintained within the first range during the first time section, changing the strength of the torque to the second torque; and
in response to identifying that the third parameter is maintained within the fourth range during the first time section and that the second parameter is maintained within the third range during the first time section, changing the strength of the torque to the second strength.

15. The method of any one of preceding claims, further comprising:
while outputting the torque having the second strength and based on the second direction, initiating output of a preset acoustic signal through a speaker included in the electronic device.
